# EUROPEAN PATENT SPECIFICATION

(11) **EP 3 029 072 B1**
(45) Date of publication and mention of the grant of the patent: **19.12.2018**
(21) Application number: 14831257.2
(22) Date of filing: 23.07.2014
(51) Int. Cl.: A61K 47/00

(54) **GLP-1 ANALOG FUSION PROTEIN AND PREPARATION METHOD AND USE THEREOF**
GLP-1-ANALOG-FUSIONSPROTEINE SOWIE HERSTELLUNGSVERFAHREN DAFÜR UND VERWENDUNG DAVON
PROTÉINE DE FUSION ANALOGUE GLP-1 ET SON PROCÉDÉ DE PRÉPARATION ET SON UTILISATION

(30) Priority: 01.08.2013 CN 201310331182
(43) Date of publication of application: 08.06.2016
(73) Proprietor: Jiangsu T-MAB Biopharma Co., Ltd., Taizhou, Jiangsu 225300 (CN)
(72) Inventor: HUANG, Yanshan, Taizhou Jiangsu 225300 (CN); YANG, Zhiyu, Taizhou Jiangsu 225300 (CN); XU, Zhengxue, Taizhou Jiangsu 225300 (CN); QIU, Jiwan, Taizhou Jiangsu 225300 (CN)
(74) Representative: Dr. Gassner & Partner mbB
(86) International application number: PCT/CN2014/082798
(87) International publication number: WO 2015/014228

(56) References cited:
- EP-A2- 1 724 284
- WO-A1-2011/109784
- WO-A2-2008/019143
- WO-A2-2009/075859
- CN-A- 101 367 873
- CN-A- 101 525 386
- CN-A- 101 974 090
- CN-A- 102 690 352
- CN-A- 103 408 669
- US-A1- 2009 005 312
- HUANG, Y.S. ET AL.: 'Preparation and Characterization of a Novel Exendin-4 Human Serum Albumin Fusion Protein Expressed in Pichia Pastoris' JOURNAL OF PEPTIDE SCIENCE vol. 14, no. 5, 12 November 2007, pages 588 - 595, XP002558144
- DOU, W.F. ET AL.: 'Expression, Purification, and Characterization of Recombinant Human Serum Albumin Fusion Protein with two Human Glucagon-like Peptide-1 Mutants in Pichia Pastoris' PROTEIN EXPRESSION AND PURIFICATION vol. 61, no. 1, 10 May 2008, pages 45 - 49, XP023182965
- LONG, JUAN ET AL.: 'Expression of 2Gly-hGLP-1-HSA Fusion Protein in Pichia Pastoris' LETTERS IN BIOTECHNOLOGY vol. 19, no. 4, 31 July 2008, pages 488 - 492, XP008182787

## Description

### FIELD OF THE INVENTION

The present invention relates to a novel GLP-1 analogue fusion protein and a method for preparing the fusion protein. The GLP-1 analogue fusion protein is used for treating diabetes and various related diseases or dysfunctions.

### BACKGROUND OF THE INVENTION

Glucagon-like peptide-1 (GLP-1) and analogues thereof such as Exendin-4 are widely used for researches on treating type-2 diabetes. Since GLP-1 polypeptides are quickly inactivated in vivo by protease dipeptidyl peptidase IV (DPP-IV) and the half-life period of GLP-1 polypeptides in plasma is very short, the widespread clinical application of GLP-1 polypeptides is difficult. Since Exendin-4 is not sensitive to enzymatic degradation of DPP-IV, the stability thereof is increased, however the molecular weight is lower (4187.61D) and the in-vivo half-life period is short, two times of injection are needed every day such that the clinical use is obstructed. At present, lots of efforts are made to solve the technical problem by means such as sustained release microsphere, PEG modification, fatty acid chain modification and albumin fusion, wherein the albumin fusion technique maintains biological and curative functions of target proteins and simultaneously greatly improves the in-vivo half-life period thereof through fusion with human albumin.

Although GLP-1 preparations and derivatives thereof are realistically feasible for treating diabetes, long-term continuous administration is needed once diabetic patients are diagnosed, the diabetic patients need to accept treatment throughout the entire life and thereby the requirements on the safety, economy and use convenience of the preparations are extremely high. However, the existing GLP-1/HSA fusion preparations have very great defects.

Firstly, compared with GLP-1 molecules, the molecular weight of albumin is huge. Therefore, after the fusion of them, due to steric hindrance, GLP-1/HSA fusion proteins substantially do not have biological activity. Albugon is a new GLP-1/HSA fusion protein designed by Laurie L. Baggio, et al., which is characterized in that an additional GLP-1 molecule is inserted therebetween as a spacer. However, about only 1% of biological activity thereof is reserved. The decrease of the biological activity causes the great increase of clinical dosage (Laurie L. Baggio, Qingling Huang, Theodore J. Brown, and Daniel J. Drucker, DIABETES Vol. 53: 2492-2500 (2004)). For example, with respect to a GLP-1 analogue Byetta®, the clinical administration dosage is only 5-10 µg per time and 1-2 times per day. However, the clinical effective administration dosage of Albugon reaches 4mg per day, the mole number of which is increased by approximate 22 times. The great increase of clinical dosage causes two problems as follows: 1) potential immunogenicity risks are increased; the increase of dosage inevitably causes the increase of concentration of medicine preparations due to a limitation of administration volume, for example, the single-time dosage of the GLP-1 analogue preparation Byetta is only 5-10 µg (50 µ 1), the concentration is only 0.25mg/ml, however the clinical single-time dosage of Albugon reaches 30mg/person and the preparation concentration reaches up to 30-50mg/ml; during transportation and storage of high-concentration protein preparations, the content of protein polymers are easily increased; researches have shown that the increase of treatment protein polymers will increase immunogenicity (Anne S. De Groot and David W. Scott, Trends Immunol Vol.28 No.11:482-490); recombined protein polymers will activate B-cell hyperplasia by cross-linking B-cell receptors such that B-cell and T-cell immunity is enabled (Rosenberg, A.S. Effects of protein aggregates: an immunologic perspective. AAPS J. 8: 501-507 (2006)); in addition, the recombined protein polymers are easily phagocytized by antigen presenting cells (APCs) such that the maturity of dendritic cells (DCs) is accelerated and thereby various immune responses are stimulated (Anne S. De Groot and David W. Scott, Trends Immunol Vol.28 No.11:482-490); and therefore, the remarkable increase of the dosage of the GLP-1/HSA fusion protein preparations will inevitably cause the increase of the risk of antibody production; and 2) the GLP-1/HSA fusion protein preparations need to be prepared by using extremely complex bioengineering technologies, the cost per unit quantity of protein is high and the great increase of the administration dosage will cause that the diabetic patients cannot afford the medicine.

Secondly, since most GLP-1 sequences are irregular and curly and are easily degraded due to attack by protease, an additional added second GLP-1 causes that Albugon is more easily attacked by protease and become instable. The instability shows defects in two aspects as follows: 1) when Albugon is recombined and expressed, regardless of a low-cost yeast expression system or a high-cost mammalian cell expression system, the GLP-1/HSA fusion protein secreted in culture supernatant is easily degraded by protease, and the degration not only lead to the decrease of the expression level, but also lead to the production of lots of non-uniform enzymatic hydrolysates, such that the final products are caused to be not uniform; and 2) after Albugon is injected in vivo, Albugon is easily degraded by protease and becomes ineffective during in-vivo circulation.

In addition, due to a limitation of product stability, at present all such products need to be stored and transported at low temperature and thereby the products are extremely inconvenient to carry by diabetic patients during outgoing and traveling.

From EP 1 724 284 A2 GLP-1 fusion proteins are known. These fusion proteins comprise a first polypeptide with a N-terminus and a C-terminus fused to a second polypeptide with a N-terminus and a C-terminus wherein the first polypeptide is a GLP-1 compound and the second polypeptide is selected from the group consisting of human albumin, human albumin analogs and fragments of human albumin and wherein the C-terminus of the first polypeptide is fused to the N-terminus of the second polypeptide via a peptide linker.

### SUMMARY OF THE INVENTION

The purpose of the present invention is to overcome the defects in the prior art, design and prepare a novel GLP-1 analogue fusion protein, which consists of three regions as follows: GLP-1 analogue-linker peptide-HSA (Human Serum Albumin). Compared with the existing products, the remarkable advantages of this fusion protein are as following:
1. The thermal stability is better, the fusion protein can be stored for a long term at room temperature without causing the activity to be decreased, and the fusion protein can be conveniently carried with and used by patients.
2. The protease-resistant stability is better, the stability in fermented supernatant and in vivo is more than 3 times of that of the existing fusion protein and the industrial preparation is facilitated.
3. The biological activity is higher and the biological activity thereof is more than 10 times of that of the existing fusion protein.

Compounds which contain GLP-1 analogues prepared by adopting the present invention have the advantages of very low production cost, higher biological activity and better in-vivo and in-vitro stability, and thereby the compounds are expected to become a kind of better diabetes treatment medicines.

In a first aspect, the present invention discloses a novel GLP-1 analogue fusion protein, a structure of which is GLP-1 analogue- linker peptide-human serum albumin (HSA).

In the GLP-1 analogue fusion protein disclosed by the present invention, the first region in the structure thereof is a GLP-1 analogue, wherein a sequence thereof is as shown by SEQ ID NO. 1 : HGEGTFTSDVSSYLEEQAAKEFIAWLVK; further, the GLP-1 analogue can also comprises 2 or 3 repetitive sequences of GLP-1 or analogues thereof; and further, the first region can also be a homolog Exendin-4 with similar functions to GLP-1.

After natural GLP-1 is processed in vivo, first 6 amino acids of a mature peptide molecule are cut off. Therefore, according to a habit in the art, a first amino acid of GLP-1 is designated as No.7. As shown in SEQ ID NO. 1, all amino acids in the polypeptide are continuously numbered. For example, a 7th site is a histidine and an 8th site is a glycine. Non-conservative positions in the GLP-1 sequence can be replaced by other amino acids without changing the activity thereof. For example, Gly8→Ala, Ser or Cys, Glu9→Asp, Gly, Ser, Cys, Thr, Asp, Gln, Tyr, Ala, Val, Ile, Leu, Met or Phe; Gly10→Ser, Cys, Thr, Asp, Glu, Tyr, Ala, Val, Ile, Leu, Met or Phe, Asp1S→Glu, Va116→Leu or Tyr; Ser18→Lys, Glu21→Asp, Ala24→Arg; Lys26→Gly, Ser, Cys, Thr, Asp, Glu, Tyr, Ala, Val, Ile, Leu, Met, Phe, Arg; Lys34→Gly, Ser, Cys, Thr, Asp, Glu, Tyr, Ala, Val, Ile, Leu, Met, Phe, Arg. C-terminal of GLP-1 can be in a deficiency of 1, 2 or 3 amino acids (Wolfgang Glaesner et al., U.S. Patent US7452966).

In the GLP-1 analogue fusion protein, the second region in the structure thereof is a connecting peptide. An N-terminal of the linker peptide is connected with a C-terminal of the first region through a peptide bond, and a C-terminal of the linker peptide is connected with an N-terminal of the HSA through a peptide bond.

Further, the sequence of the linker peptide is selected from:
a) GGGSSPPPGGGGSS (SEQ ID NO.11)
b) GGGSSGGGSSPPPAGGGSSGGGSS (SEQ ID NO.12)
c) GGGAPPPPPPPPPPSSGGG (SEQ ID NO.13)
d) AGGGAAGGGSSGGGPPPPPGGGGS (SEQ ID NO.14)
e) GGSSGAPPPPGGGGS (SEQ ID NO.15)
f) GGGSSGAPPPSGGGGSGGGGSGGGGS (SEQ ID NO.16)

In the GLP-1 analogue fusion protein, a third region in the structure thereof is human serum albumin (HSA). A sequence thereof is as shown by SEQ ID NO. 2. Non-conservative positions in the HSA sequence can be replaced by other amino acids without changing the activity thereof, such as Cys34→Ser, Leu407→Ala, Leu408→Val, Val409→Ala, Arg410→Ala, Lys413→Gln (Plumridge et al., International Patent WO2011051489).
SEQ ID NO. 2:

An amino acid sequence of the GLP-1 analogue fusion protein may be selected from SEQ ID NO. 3-5.
a) SEQ ID NO. 3:
b)SEQ ID NO. 4:
c) SEQ ID NO. 5:

In a second aspect, the present invention discloses a polynucleotide coding the GLP-1 analogue fusion protein.

In preferred embodiments of the present invention, a nucleotide coding sequence of the GLP-1 analogue fusion protein is SEQ ID NO. 10 and a corresponding protein sequence thereof is SEQ ID NO. 5. The nucleotide coding sequence of the GLP-1 analogue fusion protein disclosed by the present invention can also be SEQ ID NO. 8 and the corresponding protein sequence thereof is SEQ ID NO. 3; or the nucleotide coding sequence is SEQ ID NO. 9 and the corresponding protein sequence thereof is SEQ ID NO. 4.
SEQ ID NO. 8: nucleotide coding sequence of GLP-1 analogue fusion protein
SEQ ID NO. 9: nucleotide coding sequence of GLP-1 analogue fusion protein
SEQ ID NO. 10: nucleotide coding sequence of GLP-1 analogue fusion protein

The nucleotide sequence coding the GLP-1 analogue fusion protein can be prepared through any proper techniques well-known by one skilled in the art, including, but not limited to, recombinant DNA technique, chemical synthesis and the like; and as well, firstly a nucleotide sequence having a GLP-1 amino acid sequence can be synthesized and then sequences are interposed, replaced and removed through site-directed mutation, directed mutagenesis or other techniques well-known in the art to obtain the needed nucleotide sequence.

The nucleotide sequence coding carrier protein can be prepared through any proper techniques well-known by one skilled in the art. In one specific embodiment of the present invention, the nucleotide sequence of the carrier protein is a nucleotide sequence coding HSA or at least maintains 95% of consistency with the nucleotide sequence coding HSA.

For a technique of fusion between the nucleotide sequence coding the GLP-1 analogue and nucleotide sequence coding the carrier protein, see general description in the art, such as Molecular Cloning (J. Sambrook et al., Science Press, 1995).

In a third aspect, the present invention discloses a method for preparing the foresaid fusion protein. The method comprises the following steps: constructing an expression vector containing a gene sequence of the fusion protein, then transforming the expression vector containing the gene sequence of the fusion protein to a host cell for induced expression, and separating and obtaining the fusion protein from expression products.

The expression vector for constructing the gene sequence containing the fusion protein can be obtained by firstly synthesizing the nucleotide sequence coding the GLP-1 analogue, then fusing the nucleotide sequence with the nucleotide sequence coding the HSA and finally constructing to a proper expression vector.

The gene sequence expressing the GLP-1 analogue fusion protein can be expressed through expression systems well-known by one skilled in the art, including, but not limited to, bacteria transformed by using vectors such as recombinant phages and plasmids, yeast transformed by using yeast expression vectors, filamentous fungi transformed by using fungus vectors, insect cells and animal cells infected by using virus vectors and the like. In one specific embodiment of the present invention, the expression system selects and uses *Pichia pastoris* secretion expression. *Pichia pastoris* is high in expression level and low in cost and has the advantages of protein processing, folding and posttranslational modification of a eukaryotic expression system. During actual production, cells can be cultured through a shake flask in a laboratory or can be cultured through fermentation in a fermentation tank (including continuous, batch-to-batch, fed-batch and solid state fermentation).

The fusion protein which is secreted into culture medium can be purified through methods well-known by one skilled in the art, including, but not limited to, ultrafiltration, ammonium sulfate precipitation, acetone precipitation, ion exchange chromatography, hydrophobic chromatography, reversed phase chromatography, molecular sieve chromatography and the like. In one specific embodiment of the present invention, the inventor adopts a three-step chromatographic means which joints affinity chromatography, hydrophobic chromatography and ion exchange chromatography to enable the fusion protein to be purified uniformly.

In a fourth aspect, the present invention discloses the GLP-1 analogue fusion protein for use as a medicament.

In a fifth aspect, the present invention discloses a pharmaceutical composition containing the GLP-1 analogue fusion protein and at least one pharmaceutically acceptable carrier or excipient.

The pharmaceutical composition is mainly used for treating diabetes and related diseases.

In a sixth aspect, the present invention discloses a pharmaceutical composition for use in the treatment of diabetes and diabetes-related diseases, containing the GLP-1 analogue fusion protein and at least one pharmaceutically acceptable carrier or excipient.

The related diseases include type-2 diabetes, type-1 diabetes, obesity, serious cardiovascular events of patients suffering from type-2 diabetes and other serious complications (Madsbad S, Kielgast U, Asmar M, et al. Diabetes Obes Metab. 2011 May;13(5):394-407;Issa CM, Azar ST.Curr Diab Rep,2012 Oct;12(5):560-567; Neff LM, Kushner RF. Diabetes Metab Syndr Obes,2010 Jul 20;3:263-273;Sivertsen J, Rosenmeier J, Holst JJ,et al. Nat Rev Cardiol,2012 Jan 31;9(4):209-222) .

Indolent inorganic or organic carriers well-known by one skilled in the art include (but not limited to) saccharides and derivatives thereof, amino acids or derivatives thereof, surfactants, vegetable oil, wax, fat and polyhydroxy compounds such as polyethylene glycol, alcohols, glycerol, various preservatives, antioxidants, stabilizers, salts, buffer solution, water and the like can also be added therein, and these substances are used for improving the stability of the composition or improving the activity or biological effectiveness thereof according to the needs.

The pharmaceutical composition disclosed by the present invention can be prepared by adopting techniques well-known by one skilled in the art, including liquid or gel, freeze-drying or other forms, so as to produce medicines which are stable during storage and are suitable for administration to human or animals.

In a seventh aspect, the present invention discloses the GLP-1 analogue fusion protein for use in the treatment of diabetes and diabetes-related diseases.

For the foresaid fusion protein for use in the treatment of patients suffering from non-insulin dependent or insulin dependent diabetes, obesity and various other diseases , a reference can be made to the existing GLP-1 medicine preparations such as Byetta® (GLP-1 analogue peptide), Albugon® (GLP-1/HSA fusion protein) and Dulaglutide® (GLP-1/Fc fusion protein, and the dosage range thereof is 0.05-1mg/kg.

The protein disclosed by the present invention can be administrated solely, administrated by means of various combinations or administrated together with other treatment preparations.

In the present invention, the following abbreviations are used:
GLP-1 (glucagon like protein-1); HSA (human serum albumin)

### DESCRIPTION OF THE DRAWINGS

Fig. 1 illustrates an SDS-PAGEof the expression of GLP-1 analogue fusion proteins with different structures, wherein lanes 1-9 respectively are expression results of fusion proteins with sequences No.1-9.
Figs. 2A-D illustrate results of a pharmacodynamic test of a GLP-1 analogue fusion protein after single-dose subcutaneous injection to a normal rhesus monkey, wherein

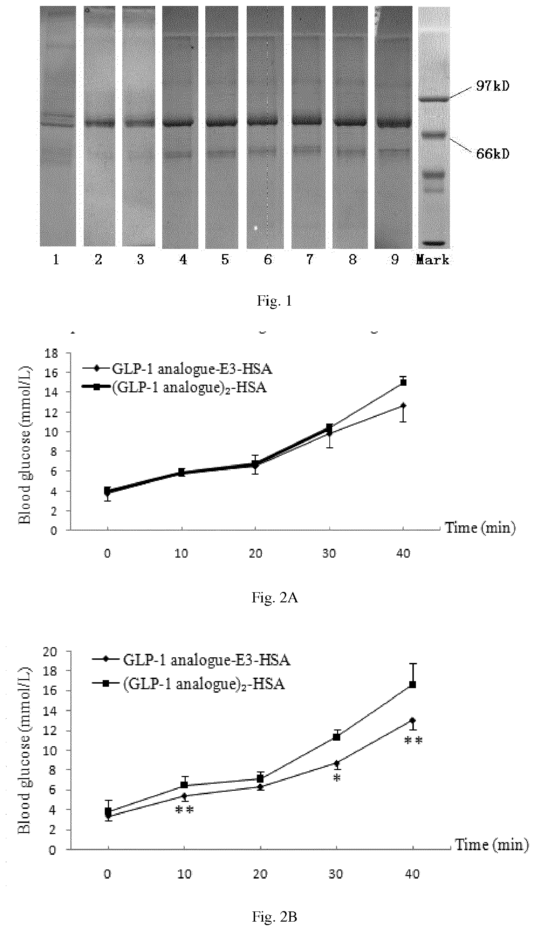

Fig. 2A illustrates blood glucose levels of a rhesus monkey during graded glucose infusion after 1 day after subcutaneous injection of GLP-1-E3-HSA.
Fig. 2B illustrates blood glucose levels of a rhesus monkey during graded glucose infusion after 4 days after subcutaneous injection of GLP-1-E3-HSA.

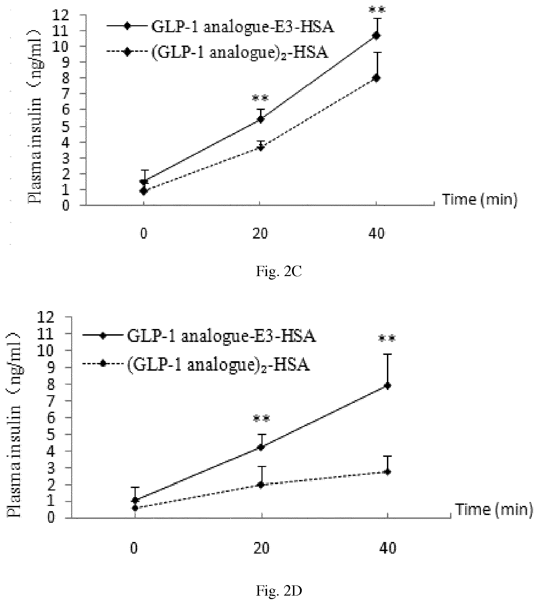

Fig. 2C illustrates insulin levels of a rhesus monkey during graded glucose infusion after 1 day after subcutaneous injection of GLP-1-E3-HSA.
Fig. 2D illustrates insulin levels of a rhesus monkey during graded glucose infusion after 4 day after subcutaneous injection of GLP-1-E3-HSA.

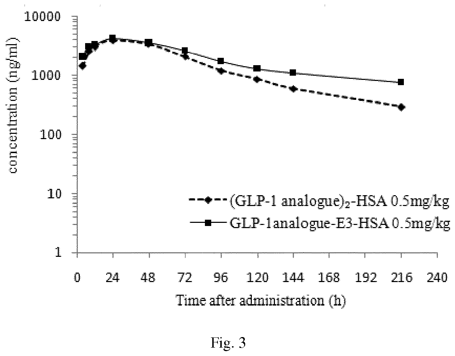

Fig. 3 illustrates a concentration-time curve chart after single-dose administration to a rhesus monkey.

### DESCRIPTION OF SEQUENCES:

SEQ ID NO. 1: amino acid sequence of GLP-1 analogue
SEQ ID NO. 2: amino acid sequence of HSA
SEQ ID NO. 3: amino acid sequence of GLP-1 analogue fusion protein
SEQ ID NO. 4: amino acid sequence of GLP-1 analogue fusion protein
SEQ ID NO. 5: amino acid sequence of GLP-1 analogue fusion protein
SEQ ID NO. 6: nucleotide coding sequence of GLP-1 analogue
SEQ ID NO. 7: nucleotide coding sequence of HSA
SEQ ID NO. 8: nucleotide coding sequence of GLP-1 analogue fusion protein
SEQ ID NO. 9: nucleotide coding sequence of GLP-1 analogue fusion protein
SEQ ID NO. 1 0: nucleotide coding sequence of GLP-1 analogue fusion protein

### DESCRIPTION OF THE EMBODIMENTS

The present invention will be described below through specific embodiments. One skilled in the art can easily understand other advantages and efficacies of the present invention according to the contents disclosed by the description. The present invention can also be implemented or applied through other different specific embodiments. Various modifications or changes can be made to all details in the description based on different points of view and applications without departing from the spirit of the present invention.

Unless otherwise stated, experiment methods, detection methods, preparation methods disclosed by the present invention adopt conventional molecular biology, biochemistry, chromatin structure and analysis, analytical chemistry, cell culture, recombinant DNA techniques in the art and conventional techniques in related arts.

### Embodiment 1: construction of recombinant fusion protein expression plasmid

Nucleotide coding sequence of GLP-1 analogue (SEQ ID NO. 6):

### 1.1 (GLP-1 analogue)₂ gene segment with an HSA fusion segment at 3'-terminal:

An oligonucleotide sequence (SEQ ID NO. 17) as follow was artificially synthesized: wherein the single line marked part is a (GLP-1 analogue)₂ gene sequence and the other part is an HSAN-terminal coding sequence.

### 1.2 GLP-1 analogue-(Gly₄Ser)₃ gene segment with an HSA fusion segment at 3'-terminal:

An oligonucleotide sequence (SEQ ID NO. 18) as follow was artificially synthesized: wherein the single line marked part is a GLP-1 analogue-(Gly₄Ser)₃ gene sequence and the other part is an HSA N-terminal coding sequence.

### 1.3 GLP-1 analogue-(Gly₄Ser)₄ gene segment with an HSA fusion segment at 3'-terminal:

An oligonucleotide sequence (SEQ ID NO. 19) as follow was artificially synthesized: wherein the single line marked part is a GLP-1 analogue-(Gly₄Ser)₄ gene sequence and the other part is an HSA N-terminal coding sequence.

### 1.4 GLP-1 analogue-El gene segment with an HSA fusion segment at 3'-terminal:

An oligonucleotide sequence (SEQ ID NO. 20) as follow was artificially synthesized: wherein the single line marked part is a GLP-1 analogue-El gene sequence and the other part is an HSA N-terminal coding sequence.

### 1.5 GLP-1 analogue-E2 gene segment with an HSA fusion segment at 3'-terminal:

An oligonucleotide sequence (SEQ ID NO. 21) as follow was artificially synthesized: wherein the single line marked part is a GLP-1 analogue-E2 gene sequence and the other part is an HSA N-terminal coding sequence.

### 1.6 GLP-1 analogue-E3 gene segment with an HSA fusion segment at 3'-terminal:

An oligonucleotide sequence (SEQ ID NO. 22) as follow was artificially synthesized: wherein the single line marked part is a GLP-1 analogue-E3 gene sequence and the other part is an HSA N-terminal coding sequence.

### 1.7 GLP-1 analogue-E4 gene segment with an HSA fusion segment at 3'-terminal:

An oligonucleotide sequence (SEQ ID NO. 23) as follow was artificially synthesized: wherein the single line marked part is a GLP-1 analogue-E4 gene sequence and the other part is an HSA N-terminal coding sequence.

### 1.8 GLP-1 analogue-E5 gene segment with an HSA fusion segment at 3'-terminal:

An oligonucleotide sequence (SEQ ID NO. 24) as follow was artificially synthesized: wherein the single line marked part is a GLP-1 analogue-E5 gene sequence and the other part is an HSA N-terminal coding sequence.

### 1.9 GLP-1 analogue-E6 gene segment with an HSA fusion segment at 3'-terminal:

An oligonucleotide sequence (SEQ ID NO. 25) as follow was artificially synthesized: wherein the single line marked part is a GLP-1 analogue-E6 gene sequence and the other part is an HSA N-terminal coding sequence.
E1: GGGSSPPPGGGGSS (SEQ ID NO.11)
E2: GGGSSGGGSSPPPAGGGSSGGGSS (SEQ ID NO.12)
E3: GGGAPPPPPPPPPPSSGGG (SEQ ID NO. 13)
E4: AGGGAAGGGSSGGGPPPPPGGGGS (SEQ ID NO.14)
E5: GGSSGAPPPPGGGGS (SEQ ID NO. 15)
E6: GGGSSGAPPPSGGGGSGGGGSGGGGS (SEQ ID NO. 16)

Notes: (Xaa)x-(Pro)y-(Xaa)z, wherein Xaa is one or any combination of a plurality of G, A and S, x, z≥3, 26≥x+y+z≥14, 10≥y≥3 and 1≥y/(x+z)≥0.13. An N-terminal of the connecting peptide is connected with a C-terminal of the first region through a peptide bond, and a C-terminal of the connecting peptide is connected with an N-terminal of the HSA through a peptide bond.

| Enhancement area | X | Y | Z | X+Y+Z | Y/X+Z |
|---|---|---|---|---|---|
| E1 | 5 | 3 | 6 | 14 | 0.272727 |
| E2 | 10 | 3 | 11 | 24 | 0.142857 |
| E3 | 4 | 10 | 7 | 21 | 0.909091 |
| E4 | 14 | 5 | 5 | 24 | 0.263158 |
| E5 | 6 | 4 | 5 | 15 | 0.363636 |
| E6 | 7 | 3 | 16 | 26 | 0.130435 |

### 2. Amplification of HSA gene

Nucleotide coding sequence of HSA (SEQ ID NO. 7):

### Primer design:

GLP-1/P1 (SEQ ID NO. 26): 5' -TCTCTCGAGAAAAGACACGGCGAAGGGACCTTTACCA GTG-3' (XhoI enzyme restriction site)
HSA/P1 (SEQ ID NO. 27): 5' -GATGCACACAAGAGTGAGG-3'
HSA/P2 (SEQ ID NO. 28): 5' -TTAGCGGCCGCTTATAAGCCTAAGGCAGCTTG-3' -(NotI enzyme restriction site)

A Human Serum Albumin/HSA/ALB Gene cDNA Clone/ORF Clone gene (Sino Biological Inc.) was used as a template, HSA/P1 and HSA/P2 were used as primers, an HSA segment was amplified, and a PCR system included 0.5µl of template, 1µl of 25µmol/L HSA/P1 and HSA/P2 respectively, 4 µl of 2mmol/L dNTP, 10µl of 5xPS reaction buffer solution, 2.5U of PrimerStar DNA polymerase and ddH₂O added to 50µl.

PCR conditions included denaturation for 10min at 98 °C and 1min 48sec at 68 °C, 25 cycles and then heat preservation at 4 °C. For PCR products, bands with molecular weight of about 1750bp were recovered through gel extraction by using agarose gel electrophoresis.

### 3. Amplification of fusion gene

### 3.1 Amplification of (GLP-1 analogue)₂-HSA fusion gene

Mixture of (GLP-1 analogue)₂ gene segments and PCR products of HSA mixed by equal mole was used as a template, GLP-1/P1 and HSA/P2 were used as primers, (GLP-1 analogue)₂-HSA was amplified, and a PCR system included 0.5µl of template, 1µl of 25µmol/L GLP-1/P1 and HSA/P2 respectively, 4 µl of 2mmol/L dNTP, 10µl of 5xPS reaction buffer solution, 2.5U of PrimerStar DNA polymerase and ddH₂O added to 50µl. PCR conditions included 10sec at 98 °C and 2min 30sec at 68 °C, 25 cycles and then heat preservation at 4 °C. For PCR products, bands with molecular weight of about 1950bp were recovered through gel extraction by using agarose gel electrophoresis.

### 3.2 Amplification of GLP-1 analogue-(Gly₄Ser)₃-HSA fusion gene

Mixture of GLP-1 analogue-(Gly₄Ser)₃ gene segments and PCR products of HSA mixed by equal mole was used as a template, a PCR system and PCR conditions were the same as 3.1, and for PCR products, bands with molecular weight of about 1930bp were recovered through gel extraction by using agarose gel electrophoresis.

### 3.3 Amplification of GLP-1 analogue-(Gly₄Ser)₄-HSA fusion gene

Mixture of GLP-1 analogue-(Gly₄Ser)₄ gene segments and PCR products of HSA mixed by equal mole was used as a template, a PCR system and PCR conditions were the same as 3.1, and for PCR products, bands with molecular weight of about 1950bp were recovered through gel extraction by using agarose gel electrophoresis.

### 3.4 Amplification of GLP-1 analogue-El-HSA fusion gene

Mixture of GLP-1 analogue-E1 gene segments and PCR products of HSA mixed by equal mole was used as a template, a PCR system and PCR conditions were the same as 3.1, and for PCR products, bands with molecular weight of about 1930bp were recovered through gel extraction by using agarose gel electrophoresis.

### 3.5 Amplification of GLP-1 analogue-E2-HSA fusion gene

Mixture of GLP-1 analogue-E2 gene segments and PCR products of HSA mixed by equal mole was used as a template, a PCR system and PCR conditions were the same as 3.1, and for PCR products, bands with molecular weight of about 1960bp were recovered through gel extraction by using agarose gel electrophoresis.

### 3.6 Amplification of GLP-1 analogue-E3-HSA fusion gene

Mixture of GLP-1 analogue-E3 gene segments and PCR products of HSA mixed by equal mole was used as a template, a PCR system and PCR conditions were the same as 3.1, and for PCR products, bands with molecular weight of about 1940bp were recovered through gel extraction by using agarose gel electrophoresis.

### 3.7 Amplification of GLP-1 analogue-E4-HSA fusion gene

Mixture of GLP-1 analogue-E4 gene segments and PCR products of HSA mixed by equal mole was used as a template, a PCR system and PCR conditions were the same as 3.1, and for PCR products, bands with molecular weight of about 1960bp were recovered through gel extraction by using agarose gel electrophoresis.

### 3.8 Amplification of GLP-1 analogue-E5-HSA fusion gene

Mixture of GLP-1 analogue-E5 gene segments and PCR products of HSA mixed by equal mole was used as a template, a PCR system and PCR conditions were the same as 3.1, and for PCR products, bands with molecular weight of about 1930bp were recovered through gel extraction by using agarose gel electrophoresis.

### 3.9 Amplification of GLP-1 analogue-E6-HSA fusion gene

Mixture of GLP-1 analogue-E6 gene segments and PCR products of HSA mixed by equal mole was used as a template, a PCR system and PCR conditions were the same as 3.1, and for PCR products, bands with molecular weight of about 1970bp were recovered through gel extraction by using agarose gel electrophoresis.

### 4. Construction of fusion protein expression plasmid

### 4.1 Construction of (GLP-1 analogue)₂-HSA expression plasmid

Firstly XhoI and NotI double enzyme restriction was performed to an expression vector plasmid pPIC9. Specific conditions were as follows: 10µl of expression vector plasmid pPIC9; 1µl of XhoI, 1µl of NotI, and 4µl of 10x enzyme restriction buffer solution (H) (purchased from Takara); and 24µl of ddH₂O and total volume of 40µl. Similar double enzyme restriction was performed to a (GLP-1 analogue)₂-HSA segment. Reaction for 2h in a 37 °C constant-temperature water bath was performed, and linearized plasmid DNA and (GLP-1 analogue)₂-HSA gene segment were recovered through agarose gel electrophoresis. The recovered vector and gene segment were ligated to construct a fusion protein expression plasmid (GLP-1 analogue)₂-HSA/pPIC9. A ligation system was generally 10µl in volume, with the molar ratio of the vector to the gene segments being 1: (2-10), including 1µl of 10xT4 DNA ligase buffer solution, 1µl of T4 DNA ligase and sterile water added to 10µl. Ligation reaction was performed for 1h in a 16 °C constant-temperature water bath. Ligation products were transformed competent cells *E.coli Top10*, transformed clone plaques were subjected to PCR identification by using general primers 5' AOX1 and 3' AOX1 as primers, correctly identified cloned bacteria solution was delivered to GenScript Corporation and sequencing was performed by using general primers 5' AOX1 and 3' AOX1. As verified by sequencing, the expectation was met.

### 4.2 Construction of GLP-1 analogue-(Gly₄Ser)₃-HSA expression plasmid

Except the fusion protein gene segment which was replaced by a GLP-1 analogue-(Gly₄Ser)₃-HSA fusion gene, others were the same as 4.1, and as verified by sequencing, the expectation was met.

### 4.3 Construction of GLP-1 analogue-(Gly₄Ser)₄-HSA expression plasmid

Except the fusion protein gene segment which was replaced by a GLP-1 analogue-(Gly₄Ser)₄-HSA fusion gene, others were the same as 4.1, and as verified by sequencing, the expectation was met.

### 4.4 Construction of GLP-1 analogue-E1-HSA expression plasmid

Except the fusion protein gene segment which was replaced by a GLP-1 analogue-E1-HSA fusion gene, others were the same as 4.1, and as verified by sequencing, the expectation was met.

### 4.5 Construction of GLP-1 analogue-E2-HSA expression plasmid

Except the fusion protein gene segment which was replaced by a GLP-1 analogue-E2-HSA fusion gene, others were the same as 4.1, and as verified by sequencing, the expectation was met.

### 4.6 Construction of GLP-1 analogue-E3-HSA expression plasmid

Except the fusion protein gene segment which was replaced by a GLP-1 analogue-E3-HSA fusion gene, others were the same as 4.1, and as verified by sequencing, the expectation was met.

### 4.7 Construction of GLP-1 analogue-E4-HSA expression plasmid

Except the fusion protein gene segment which was replaced by a GLP-1 analogue-E4-HSA fusion gene, others were the same as 4.1, and as verified by sequencing, the expectation was met.

### 4.8 Construction of GLP-1 analogue-E5-HSA expression plasmid

Except the fusion protein gene segment which was replaced by a GLP-1 analogue-E5-HSA fusion gene, others were the same as 4.1, and as verified by sequencing, the expectation was met.

### 4.9 Construction of GLP-1 analogue-E6-HSA expression plasmid

Except the fusion protein gene segment which was replaced by a GLP-1 analogue-E6-HSA fusion gene, others were the same as 4.1, and as verified by sequencing, the expectation was met.

### Embodiment 2: construction of engineering bacteria for fusion protein expression

Clones respectively containing (GLP-1 analogue)₂-HSA/pPIC9, GLP-1 analogue-(Gly₄Ser)₃-HSA/pPIC9, GLP-1 analogue-(Gly₄Ser)₄-HSA/pPIC9, GLP-1 analogue-E1-HSA/pPIC9, GLP-1 analogue-E2-HSA/pPIC9, GLP-1 analogue-E3-HSA/pPIC9, GLP-1 analogue-E4-HSA/pPIC9, GLP-1 analogue-E5-HSA/pPIC9 and GLP-1 analogue-E6-HSA/pPIC9 expression vector plasmids were selected. The expression vector plasmids were respectively extracted, then respectively linearized by using SalI. The linearized plasmid DNA were respectively recovered through agarose gel electrophoresis, and finally were respectively transformed to *Pichia pastoris* GS115 competent cells by using an electrotransformation method. After electric shock, 1ml of 1M sorbitol solution was added to cell and mixed immediately, then the solution was transferred to a 1.5ml centrifugal tube and placed at 30 °C for 1.5h, then the cell suspension was coated on RDB selective plates with every 300µl of cell suspension per. The plates were cultured at 30 °C for culture until single colonies occurred. The positive colonies were transferred to fresh RDB plates and cultured for 24h, then single colonies, corresponding to each GLP-1 analogue fusion protein, which grown on the RDB plates were respectively selected and inoculated in 10ml of BMGY culture medium, cultured for 24h at 30 °C and 250rpm. Cell suspension was placed and the supernatant was discarded, then the cells were resuspended by using 10ml of BMMY (2% methanol). Cells were induced for 48h at 30 °C and 250rpm, then the supernatant was collected by centrifugation to detect the expression of the fusion proteins through 10% SDS-PAGE electrophoresis. The N-terminals of the fusion proteins were sequenced if the size of electrophoresis bands met the expectation, and the sequencing results which met the expectation means engineering strains with each GLP-1 analogue fusion protein were construced successfully.

Specific conditions for linearizing plasmids were as follows: 60µl of expression vector plasmid, 2.5µl of SalI, 20µl of 10 x buffer solutions (H) and added to 200µl by ddH₂O. Reaction was performed for 3h in a 37 °C constant-temperature water bath.

A specific method for preparing competent cells comprised the following steps: firstly preparing colonies, selecting yeast single colonies, inoculating the single colonies in a 50ml triangular flask containing 5ml of YPD culture medium, and performing culture at 30 °C and 250rpm overnight; then taking and inoculating 30µl of culture into a 250ml triangular flask containing 50ml of YPD culture medium, and performing culture at 30 °C and 250 rpm overnight until OD600 reached 1-1.5; precooling cell culture on ice for 10min, then performing centrifugation for 5min at 4 °C and 1500×g, discarding supernatant, and resuspending the precipitation of cells with 40ml of precooled sterile water, centrifugating, then resuspending the precipitation of cells with 25ml of precooled sterile water, recentrifugating and resuspending the precipitation of cells with 5ml of precooled 1M sorbitol solution, then recentrifugating and resuspending the precipitation of cells with 80µl of precooled 1M sorbitol solution.

A specific electrotransformation method comprised the following steps: uniformly mixing 10µl of linearized plasmids with 80µl of the competent cells, transferring the mixture to a 0.2cm ice-precooled electrotransformation cup, placing the electrotransformation cup in an ice bath for 5min and then performing electric shock by using 1500V voltage.

### Embodiment 3: preparation of GLP-1 analogue fusion proteins

Referring to Manual of Methods for Expression of Recombinant Proteins in *Pichia pastoris* (Invitrogen Corporation), strains, expressing each GLP-1 analogue fusion protein, which were obtained in embodiment 2 were inoculated in YPD culture medium. Culture was performed by shaking at 30 °C and 220-280rpm until the wet weight of the cells reached about 50g/L, the cells were inoculated into bioreaction (Biostat C10, Sartorius) by a dosage of 10%. Culture was performed for 20h at 30 °C, pH 5.0 and 30% of dissolved oxygen saturation. Then methanol was continuously fed to start induction. The dissolved oxygen saturation was controlled at 40%. The temperature was reduced to 22 °C after induction for 4h. The induction was ended after 50h and the supernatant was collected by centrifugation for 15min at 10000×g and fermented supernatant was collected.

BLUE affinity, PHE hydrophobic, DEAE ion exchange and gel exclusion four-step chromatography was adopted for purification. Firstly, the fermented supernatant was diluted by three times by using 20mM pH 7.0 sodium phosphate solution, then the solution passed through a Blue Sepharose Fast Flow (XK 50/20, GE healthcare) affinity chromatography column, balancing was performed by using PBS, and then the target protein was eluted by using 2M NaCl and 20mM pH 6.5 sodium phosphate solution. (NH₄)₂SO₄ was added into the collected protein solution to enable the final concentration to reach 0.5M, the protein solution passed through a PHE Sepharose Fast Flow (XK 50/20, GE healthcare) chromatography column, balancing was performed by using 0.6M (NH₄)₂SO₄, and then the protein was eluted by using 5mM pH 6.5 sodium phosphate buffer solution. The collected protein was diluted by two times by using 5mM pH 6.5 sodium phosphate buffer solution, then the solution passed through an ion exchange chromatography column, and the target protein was eluted directly by using PBS by adopting a DEAE Sepharose Fast Flow (XK 50/20, GE healthcare) chromatography column. Finally, desalination was performed through a Sephadex G25 coarse (XK 50/60, GE healthcare) gel chromatography column to realize displacement into 5mM pH 6.5 sodium phosphate buffer solution. The expression supernatant and the purified fusion protein were respectively analyzed by using non-reductive SDS-PAGE. As shown in Fig. 1, there was a great difference in stability of GLP-1 analogue fusion proteins with different structures during expression, wherein the stability of (GLP-1 analogue)₂-HSA is the poorest.

### Embodiment 4: in-vitro activity test

According to the literature (Zlokarnik G, Negulescu PA, Knapp TE, Mere L, Burres N, Feng L, Whitney M, Roemer K, Tsien RY. Science. 279 (5347): 84-8. (1998)), HEK-293 cells carrying with human GLP-1 receptors and CRE-Luc reporter genes were constructed, and DMEM culture containing 10% of FBS according to 50000 cells/well/200µl was used for inoculation into a Costar 96-well cell culture plate. On the second day after inoculation, culture solution was absorbed away, 50µl of serum-free DMEM culture solution of stepwise diluted GLP-1 analogue fusion proteins containing 500µM IBMX was added into each well, incubation was performed for 5-6h, then 50µl of luciferase substrate (Bright-Glo™ Luciferase Assay System, Promega, E2620) was added.Reaction was performed for 2min, then the solution was transferred to a Costar 96-well all-white micro-well plate. Fluorescence values were determined on a multifunctional ELISA microplate reader (SpectraMax M5 system, Molecular Device). A dose-response curve was depicted according to the fluorescence values and an EC₅₀ value was determined. By taking the activity of (GLP-1 analogue)₂-HSA as 100%, relative activity of each fusion protein was calculated. Results were as shown in Table 1. The in-vitro activity of Gly₄Ser as a connecting peptide was substantially similar to that of a GLP-1 analogues as a connecting peptide; and however, when a segment of sequences (E1-E6) according to claim 1 was inserted between the GLP-1 analogue and HSA, the in-vitro activity of the fusion protein was improved by about 7-10 times.

**Table 1**

| | Fusion protein | Relative activity (%) | Standard deviation |
|---|---|---|---|
| 1 | (GLP-1 analogue)₂-HSA | 100 | 17 |
| 2 | GLP-1 analogue-(Gly₄Ser)₃-HSA | 103 | 18 |
| 3 | GLP-1 analogue-(Gly₄Ser)₄-HSA | 108 | 22 |
| 4 | GLP-1 analogue-E1-HSA | 752 | 98 |
| 5 | GLP-1 analogue-E2-HSA | 823 | 124 |
| 6 | GLP-1 analogue-E3-HSA | 1108 | 89 |
| 7 | GLP-1 analogue-E4-HSA | 957 | 141 |
| 8 | GLP-1 analogue-E5-HSA | 1003 | 125 |
| 9 | GLP-1 analogue-E6-HSA | 763 | 99 |

### Embodiment 5: in-vitro stability analysis

High-purity GLP-1 analogue fusion protein stock solution was taken, proper amounts of sodium chloride, disodium hydrogen phosphate and sodium dihydrogen phosphate were added, pH was regulated to 7.4 by using sodium hydroxide or hydrochloric acid, and then water for injection was added to enable 1ml of solution to contain 5.0mg of GLP-1 analogue protein, 9mg of sodium chloride and 20µmol of phosphate. Bacteria were removed by using a 0.22µm PVDF or PES filter membrane. The solution was aseptically packaged in a penicillin bottle under a class-100 environment. The sample was stored in a stability test box at 25 °C, and samples were respectively taken at the 0^{th}, 1^{st} and 3^{rd} month and was stored in a -70 °C refrigerator for detection. All samples to be analyzed were combined and SDS-PAGE purity and cell biological activity were detected. The method for detecting the SDS-PAGE purity was as described in embodiment 1 and the loading amount of the sample to be detected was 10ug. In addition, 1ug, 0.5ug, 0.2ug, 0.1ug and 0.05ug of self-control were loaded. Optical density scanning was performed to obtain a standard curve, the percentage content of each impure protein was calculated and finally the purity of the fusion protein was calculated. The method for determining in-vitro activity was as described in embodiment 4, and the activity of each sample at the zero month was taken as 100%. Before activity determination, the sample was separated by using a Superdex 75 10/30 molecular sieve column (GE Healthcare) to remove degraded segments with molecular weight which was smaller than 10000Da. So as to avoid the disturbance thereof to the activity determination. Results were as shown in Table 2. When the GLP-1 analogue was inserted as a connecting peptide between the GLP- analogue and HSA, the activity preservation rate was the poorest and the fusion protein was the most instable.

**Table 2**

| | Fusion protein | SDS-PAGE purity (%) | | | Activity preservation rate (%) | | |
|---|---|---|---|---|---|---|---|
| | | 0 | 1 | 3 | 0 | 1 | 3 |
| 1 | (GLP-1 analogue)₂-HSA | 97.1 | 65.3 | 34.3 | 100 | 45.4 | 13.5 |
| 2 | GLP-1 analogue-(Gly₄Ser)₃-HSA | 97.3 | 84.3 | 67.3 | 100 | 79.0 | 47.2 |
| 3 | GLP-1 analogue-(Gly₄Ser)₄-HSA | 98.0 | 89.4 | 66.0 | 100 | 85.5 | 45.3 |
| 4 | GLP-1 analogue-E1-HSA | 97.5 | 93.4 | 77.9 | 100 | 97.6 | 60.6 |
| 5 | GLP-1 analogue-E2-HSA | 98.3 | 94.3 | 72.8 | 100 | 93.4 | 57.9 |
| 6 | GLP-1 analogue-E3-HSA | 97.9 | 95.1 | 77.3 | 100 | 91.7 | 49.8 |
| 7 | GLP-1 analogue-E4-HSA | 97.5 | 94.8 | 75.2 | 100 | 96.2 | 55.6 |
| 8 | GLP-1 analogue-E5-HSA | 98.4 | 95.7 | 81.2 | 100 | 93.3 | 47.2 |
| 9 | GLP-1 analogue-E6-HSA | 98.2 | 96.7 | 80.1 | 100 | 91.7 | 53.9 |

### Embodiment 6: serum stability analysis

Purified high-purity GLP-1 analogue fusion protein stock solution was taken and added into monkey serum according to a volume ratio of 1:25, filtration was performed to remove bacteria, the solution was aseptically packaged in a penicillin bottle and incubation was performed at 37 °C. Samples were taken at the 0^{th}, 15^{th} and 30^{th} day and stored in a -70 °C refrigerator for detection. All samples to be analyzed were combined, and fusion protein concentration was determined through a sandwich ELISA method by using Anti-GLP-1 monoclonal antibodies (Antibodyshop) as capture antibodies and Goat anti-Human Albumin-HRP (Bethyl Laboratories) as detection antibodies. Since the capture antibodies were bound to the portion of the GLP-1 analogue of the fusion protein and the detection antibodies were bound to the portion of the albumin, the determined fusion protein concentration was positively correlated with the content of the undegraded portion. Results were as shown in Table 3. After 30 days, most (GLP-1 analogue)₂-HSA in the monkey serum had already been degraded and about 40% of other samples were reserved.

**Table 3 Change situation of content of fusion protein in serum with time**

| | Fusion protein | Fusion protein content (%) | | |
|---|---|---|---|---|
| | | 0^{th} day | 15^{th} day | 30^{th} day |
| 1 | (GLP-1 analogue)₂-HSA | 100 | 37.4 | 17.8 |
| 2 | GLP-1 analogue-(Gly₄Ser)₃-HSA | 100 | 65.4 | 34.3 |
| 3 | GLP-1 analogue-(Gly₄Ser)₄-HSA | 100 | 69.3 | 37.6 |
| 4 | GLP-1 analogue-E1-HSA | 100 | 74.5 | 44.9 |
| 5 | GLP-1 analogue-E2-HSA | 100 | 72.0 | 41.2 |
| 6 | GLP-1 analogue-E3-HSA | 100 | 66.3 | 45.5 |
| 7 | GLP-1 analogue-E4-HSA | 100 | 73.2 | 48.2 |
| 8 | GLP-1 analogue-E5-HSA | 100 | 75.4 | 55.3 |
| 9 | GLP-1 analogue-E6-HSA | 100 | 76.9 | 45.1 |

| | | | | |
|---|---|---|---|---|
| Note: the concentration determined on the 0^{th} day was taken as 100%. | | | | |

### Embodiment 7: mouse intraperitoneal glucose tolerance test

Totally 32 KM mice including 16 female mice and 16 male mice were taken and fed no food but water only overnight for 18h, and then subcutaneous injection of 1.0 mg/kg HSA (control group), (GLP-1 analogue)₂-HSA, GLP-1 analogue-E3-HSA and GLP-1 analogue-E6-HSA was performed. After 1 hour and 8 hours after administration, intraperitoneal glucose tolerance tests (IPGTT) were respectively performed, intraperitoneal injection of 1.5 g/kg glucose was performed, and blood was taken before (t=0) glucose injection and after 10min, 20min, 30min, 60min, 90min and 120min after glucose injection to determine the content of glucose in blood (YSI2700 biochemical analyzer). Compared with the control group (HSA group), the blood glucose of the mice of the (GLP-1 analogue)₂-HSA, GLP-1 analogue-E3-HSA and GLP-1 analogue-E6-HSA groups was obviously reduced, and the area under curve (AUC₀₋₁₂₀ₘᵢₙ) of blood glucose was obviously smaller than that of the control group (results were as shown in Table 4). When the IPGTT was performed after 1 hour after administration, the blood glucose levels at respective time point among the three groups were similar, the areas under curve (AUC₀₋₁₂₀ₘᵢₙ) of blood glucose were also similar, and no remarkable difference (P>0.05) existed among the groups; and however, when the IPGTT was performed after 8 hours after administration, the blood glucose levels of the mice of the GLP-1 analogue-E3-HSA and GLP-1 analogue-E6-HSA groups at 10-30min were obviously lower than that of the (GLP-1 analogue)₂-HSA group, and the AUC₀₋₁₂₀ₘᵢₙ of blood glucose was also obviously lower than that of the (GLP-1 analogue)₂-HSA group (P<0.01). The results shown that both (GLP-1 analogue)₂-HSA and GLP-1 analogue-E3-HSA could effectively reduce mice fasting blood-glucose and had a long-acting feature, but compared with the (GLP-1 analogue)₂-HSA group, the GLP-1 analogue-E3-HSA and GLP-1 analogue-E6-HSA groups had more remarkable and continuous blood glucose reducing effects.

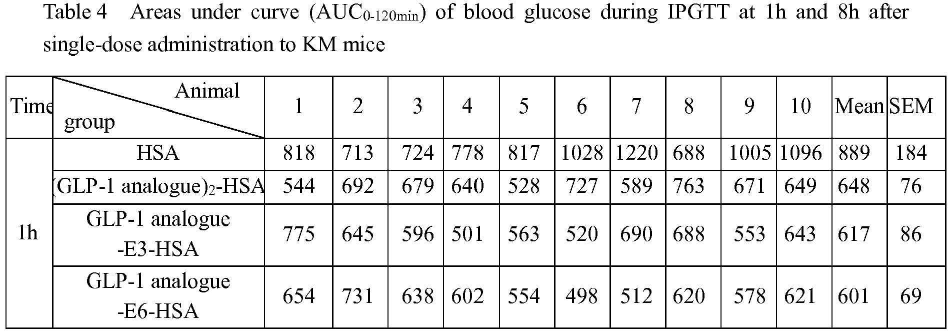

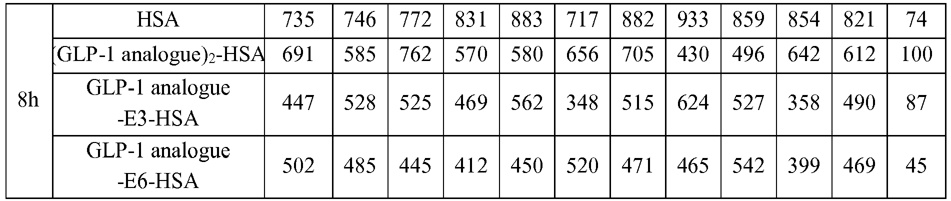

### Embodiment 8: pharmacodynamic test of GLP-1 analogue fusion protein after single-dose subcutaneous injection to normal rhesus monkey

Single-dose subcutaneous injection of 0.5 mg/kg (GLP-1 analogue)₂-HSA or GLP-1 analogue-E3-HSA was performed to a rhesus monkey, stepwise intravenous glucose tests were performed after 24h and 96h, intravenous injection of glucose solution (20% dextrose solution, 200 mg/ml) was performed continuously for 20min according to 10mg/kg/min (3.0ml/kg/h), and then glucose solution was administrated continuously for 20min according to 25 mg/kg/min (7.5 ml/kg/h). Blood was acquired after 0, 10min, 20min, 30min and 40min after glucose injection to determine blood glucose and insulin. YSI2700 biochemical analyzer was used for determining blood glucose and enzyme-linked immunosorbent assay (Insulin ELISA kit, DRG International, Inc.) was used for determining insulin. There was no remarkable difference in blood glucose between the two groups at respective time point after Id after administration (results were shown in Figs. 2A-D). There was a remarkable difference (P<0.05 or P<0.01) between the groups at 10min, 30min and 40min after 4d after administration; and there was a remarkable difference (P<0.01) in insulin between the groups at time points 20min and 40min after 1d and 4d after administration. The results shown that, compared with (GLP-1 analogue)₂-HSA, GLP-1 analogue-E3-HSA could better promote the secretion of insulin and reduce the blood glucose level in the stepwise intravenous glucose test carried out to the normal rhesus monkey.

### Embodiment 9: pharmacokinetic research after single-dose administration to crab-eating macaque

Single-dose subcutaneous injection of 0.5mg/kg (GLP-1 analogue)₂-HSA and GLP-1 analogue E3-HSA was respectively performed to crab-eating macaques, blood was respectively acquired before administration (t=0) and after 4h, 8h, 12h, 24h, 48h, 72h, 96h, 120h, 144h and 216h after administration, serum was separated, cryopreservation was performed at -80 °C and then the serum was combined and detected. Concentration of fusion protein in the serum was determined by using Anti-GLP-1 monoclonal antibodies (Antibodyshop) as capture antibodies and Goat anti-Human Albumin-HRP (Bethyl Laboratories) as detection antibodies (see Fig. 3), and pharmacokinetic parameters (see Table 5) were calculated. The research shown that the half-life period of the 0.5mg/kg GLP-1 analogue-E3-HSA in the body of the crab-eating macaque was 102h (about 4d) and the half-life period of the (GLP-1 analogue)₂-HSA was 60h (2.5d).

**Table 5**

| Parameters | (GLP-1 analogue)₂-HSA | GLP-1 analogue-E3-HSA |
|---|---|---|
| Cₘₐₓ(ng/ml) | 3954 | 4452 |
| Tₘₐₓ(h) | 24 | 24 |
| AUC_{0-∞}(ng/ml*h) | 356210 | 516613 |
| T_{1/2}(h) | 60 | 102 |
| CL(ml/h/kg) | 1.404 | 0.968 |

### Embodiment 10: immunogenicity after repetitive subcutaneous administration to crab-eating macaque

Subcutaneous injection of 1mg/kg (GLP-1 analogue)₂-HSA and GLP-1 analogue-E3-HSA was weekly performed to crab-eating macaques, and administration was continuously performed for 3 months. Blood was respectively acquired before administration (t=0) and after 1 month, 2 months and 3 months after administration, serum was separated, cryopreservation was performed at -80 °C and then the serum was combined and detected. Monkey-anti-fusion protein antibodies which were possibly produced were determined by using enzyme-linked immunosorbent assay (ELISA). Corresponding fusion proteins were used as encrusting substances, to-be-detected serum samples of different dilution were added, and the titer of the antibodies was determined by using mouse-anti-monkey IgG as detection antibodies. Simultaneously, under similar determination conditions, human serum albumin was added as antagonist into the to-be-detected serum samples (final concentration of 60µM) to further analyze the produced antibody specificity (see Table 6). Research results shown that, after repetitive administration, antibodies were produced by the both, the highest titer reached 1:6400, and the trends and titers of the antibodies produced by the both were substantially consistent. HSA was further added into serum for antagonistic analysis, results shown that the titer of the serum was obviously decreased under the existence of HSA and it indicated that the produced antibodies were substantially antagonized by HSA. Therefore, it indicated that most antibodies produced after repetitive injection of fusion protein to macaques were directed at the portion of HSA in the fusion protein and no anti-GLP-1 analogue antibodies were produced.

**Table 6**

| Fusion protein | Animal No No. | Titer of anti-fusion protein antibody | | | | Titer of anti-GLP-1 analogue antibody | | | |
|---|---|---|---|---|---|---|---|---|---|
| | | Before administration | 1 month | 2 months | 3 months | Before administration | 1 month | 2 months | 3 months |
| (GLP-1 analogue)₂-HSA | 1 | N.D. | N.D. | 1:1600 | 1:6400 | N.D. | N.D. | N.D. | 1:100 |
| | 2 | N.D. | 1:100 | 1:6400 | 1:6400 | N.D. | N.D. | N.D. | 1:200 |
| | 3 | N.D. | N.D. | 1:1600 | 1:1600 | N.D. | N.D. | N.D. | N.D. |
| GLP-1 analogue-E3 -HSA | 4 | N.D. | N.D. | N.D. | 1:100 | N.D. | N.D. | N.D. | N.D. |
| | 5 | N.D. | 1:100 | 1:6400 | 1:6400 | N.D. | N.D. | 1:100 | N.D. |
| | 6 | N.D. | N.D. | 1:1600 | 1:1600 | N.D. | N.D. | N.D. | N.D. |

| | | | | | | | | | |
|---|---|---|---|---|---|---|---|---|---|
| Note: antibody titer < 1:100 was defined as not detected (N.D.). | | | | | | | | | |

The above-mentioned embodiments are only used for exemplarily describing the principle and efficacies of the present invention instead of limiting the present invention. One skilled in the art can make modifications or changes to the above-mentioned embodiments without departing from the spirit and the range of the present invention.

## Claims

1. A GLP-1 analogue fusion protein, **characterized in that** a structure of the fusion protein is GLP-1 analogue-linker peptide-human serum albumin, the amino acid sequence of the linker peptide is any one of SEQ ID NO. 11-16, an N-terminal of the linker peptide is connected with a C-terminal of the GLP-1 analogue through a peptide bond, and a C-terminal of the linker peptide is connected with an N-terminal of the human serum albumin through a peptide bond, wherein the GLP-1 analogue is any one of follows:
a) having an amino acid sequence of SEQ ID NO. 1;
b) having an amino acid sequence of SEQ ID NO. 1 in which sequence Gly at position 2 is replaced by Ala, Ser or Cys and/or Glu at position 3 is replaced by Asp, Gly, Ser, Cys Thr, Asp, Gln, Tyr, Ala, Val, Ile, Leu, Met or Phe and/or Gly at position 4 is replaced by Ser, Cys, Thr, Asp, Glu, Tyr, Ala, Val, Ile, Leu, Met or Phe and/or Asp at position 9 is replaced by Glu and/or Val at position 10 is replaced by Leu or Tyr and/or Ser at position 12 is replaced by Lys and/or Glu at position 15 is replaced by Asp and/or Ala at position 18 is replaced by Arg and/or Lys at position 20 is replaced by Gly, Ser, Cys, Thr, Asp, Glu, Tyr, Ala, Val, Ile, Leu, Met, Phe or Arg and/or Lys at position 28 is replaced by Gly, Ser, Cys, Thr, Asp, Glu, Tyr, Ala, Val, Ile, Leu, Met, Phe or Arg without changing activity thereof;
c) comprising 2 or 3 repetitive sequences of the GLP-1 analogue of a) or b), or comprising 2 or 3 repetitive sequences of a GLP-1; and
d) being Exendin-4
or wherein an amino acid sequence of the GLP-1 analogue fusion protein is selected from SEQ ID NO. 3-5.

2. The GLP-1 analogue fusion protein according to claim 1, **characterized in that** an amino acid sequence of the human serum albumin is SEQ ID NO. 2 or the sequence of SEQ ID NO. 2 at which sequence Cys at position 34 is replaced by Ser and/or Leu at position 407 is replaced by Ala and/or Leu at position 408 is replaced by Val and/or Val at position 409 is replaced by Ala and/or Arg at position 410 is replaced by Ala and/or Lys at position 413 is replaced by Gln without changing activity thereof.

3. A polynucleotide coding the GLP-1 analogue fusion protein according to claim 1 or 2.

4. The polynucleotide according to claim 3, **characterized in that** a sequence of the polynucleotide is selected from SEQ ID NO. 8-10.

5. A method for preparing the GLP-1 analogue fusion protein according to claim 1 or 2, the method comprising the following steps: constructing an expression vector containing a gene sequence of the GLP-1 analogue fusion protein, then transforming the expression vector in a host cell for induced expression, and separating and obtaining the fusion protein from expression products.

6. The method for preparing the GLP-1 analogue fusion protein according to claim 5, **characterized in that** the expression vector is pPIC9; and the host cell is *Pichia pastoris.*

7. The method for preparing the GLP-1 analogue fusion protein according to claim 5, **characterized in that** a method for separating and obtaining the fusion protein from the expression products comprises the step of separating and obtaining the fusion protein by adopting a three-step chromatographic method which joints affinity chromatography, hydrophobic chromatography and ion exchange chromatography.

8. The GLP-1 analogue fusion protein according to claim 1 or 2 for use as a medicament.

9. A pharmaceutical composition for use in treating diabetes and diabetes-related diseases, containing the GLP-1 analogue fusion protein according to claim 1 or 2 and at least one pharmaceutically acceptable carrier or excipient.

10. A pharmaceutical composition for use in the treatment of diabetes and diabetes-related diseases, containing the GLP-1 analogue fusion protein according to claim 1 or 2 and at least one pharmaceutically acceptable carrier or excipient.

11. The GLP-1 analogue fusion protein according to claim 1 or 2 for use in the treatment of diabetes and diabetes-related diseases.

## Patentansprüche

1. GLP-1 -analoges Fusionsprotein, **dadurch gekennzeichnet, dass** eine Struktur des Fusionsproteins GLP-1-Analogon - Linkerpeptid - humanes Serumalbumin ist, wobei die Aminosäuresequenz des Linkerpeptids eine von SEQ ID NO. 11-16 ist, ein N-Terminus des Linkerpeptids mit einem C-Terminus des GLP-1-Analogons durch eine Peptidbindung verbunden ist und ein C-Terminus des Linkerpeptids mit einem N-Terminus des human Serumalbumins durch eine Peptidbindung verbunden ist, wobei das GLP-1-Analogon eines der Folgenden ist:
a) Eine Aminosäuresequenz von SEQ ID NO. 1 aufweisend,
b) eine Aminosäuresequenz von SEQ ID NO. 1 aufweisend, in welcher Sequenz Gly an Position 2 ersetzt ist durch Ala, Ser oder Cys und/oder Glu an Position 3 ersetzt ist durch Asp, Gly, Ser, Cys, Thr, Asp, Gln, Tyr, Ala, Val, Ile, Leu, Met oder Phe und/oder Gly an Position 4 ersetzt ist durch Ser, Cys, Thr, Asp, Glu, Tyr, Ala, Val, Ile, Leu, Met oder Phe und/oder Asp an Position 9 ersetzt ist durch Glu und/oder Val an Position 10 ersetzt ist durch Leu oder Tyr und/oder Ser an Position 12 ersetzt ist durch Lys und/oder Glu an Position 15 ersetzt ist durch Asp und/oder Ala an Position 18 ersetzt ist durch Arg und/oder Lys an Position 20 ersetzt ist durch Gly, Ser, Cys, Thr, Asp, Glu, Tyr, Ala, Val, Ile, Leu, Met, Phe oder Arg und/oder Lys an Position 28 ersetzt ist durch Gly, Ser, Cys, Thr, Asp, Glu, Tyr, Ala, Val, Ile, Leu, Met, Phe oder Arg ohne die Aktivität davon zu verändern,
c) 2 oder 3 repetitive Sequenzen des GLP-1 -Analogons aus a) oder b) umfassend oder 2 oder 3 repetitive Sequenzen eines GLP-1 umfassend, und
d) Exendin-4 seiend
oder wobei eine Aminosäuresequenz des GLP-1-analogen Fusionsproteins aus SEQ ID NO. 3-5 ausgewählt ist.

2. GLP-1-analoges Fusionsprotein nach Anspruch 1, **dadurch gekennzeichnet, dass** eine Aminosäuresequenz des humanen Serumalbumins SEQ ID NO. 2 oder die Sequenz von SEQ ID NO. 2 ist, bei welcher Sequenz Cys an Position 34 ersetzt ist durch Ser und/oder Leu an Position 407 ersetzt ist durch Ala und/oder Leu an Position 408 ersetzt ist durch Val und/oder Val an Position 409 ersetzt ist durch Ala und/oder Arg an Position 410 ersetzt ist durch Ala und/oder Lys an Position 413 ersetzt ist durch Gln ohne die Aktivität davon zu verändern.

3. Polynukleotid, codierend das GLP-1-analoge Fusionsprotein nach Anspruch 1 oder 2.

4. Polynukleotid nach Anspruch 3, **dadurch gekennzeichnet, dass** eine Sequenz des Polynukleotids aus SEQ ID NO. 8-10 ausgewählt ist.

5. Verfahren zum Herstellen des GLP-1-analogen Fusionsproteins nach Anspruch 1 oder 2, wobei das Verfahren die folgenden Schritte umfasst: Herstellen eines eine Gensequenz des GLP-1-analogen Fusionsproteins enthaltenden Expressionsvektors, dann Transformieren des Expressionsvektors in eine Wirtszelle zur induzierten Expression und Absondern und Erhalten des Fusionsproteins aus den Expressionsprodukten.

6. Verfahren zum Herstellen des GLP-1-analogen Fusionsproteins nach Anspruch 5, **dadurch gekennzeichnet, dass** der Expressionsvektor pPIC9 ist und die Wirtszelle *Pichia pastoris* ist.

7. Verfahren zum Herstellen des GLP-1-analogen Fusionsproteins nach Anspruch 5, **dadurch gekennzeichnet, dass** ein Verfahren zum Absondern und Erhalten des Fusionsproteins aus den Expressionsprodukten den Schritt des Absonderns und Erhaltens des Fusionsproteins durch Anwenden eines chromatographischen Drei-Schritt-Verfahrens, welches Affinitätschromatographie, hydrophobe Chromatographie und lonenaustauschchromatographie vereint, umfasst.

8. GLP-1-analoges Fusionsprotein nach Anspruch 1 oder 2 zur Verwendung als ein Medikament.

9. Pharmazeutische Zusammensetzung zur Verwendung beim Behandeln von Diabetes und mit Diabetes in Verbindung stehenden Erkrankungen, enthaltend das GLP-1-analoge Fusionsprotein nach Anspruch 1 oder 2 und mindestens einen pharmazeutisch akzeptablen Träger oder Hilfsstoff.

10. Pharmazeutische Zusammensetzung zur Verwendung bei der Behandlung von Diabetes und mit Diabetes in Verbindung stehenden Erkrankungen, enthaltend das GLP-1-analoge Fusionsprotein nach Anspruch 1 oder 2 und mindestens einen pharmazeutisch akzeptablen Träger oder Hilfsstoff.

11. GLP-1 -analoges Fusionsprotein nach Anspruch 1 oder 2 zur Verwendung bei der Behandlung von Diabetes und mit Diabetes in Verbindung stehenden Erkrankungen.

## Revendications

1. Protéine de fusion d'analogue de GLP-1, **caractérisée en ce qu'**une structure de la protéine de fusion est analogue de GLP-1-peptide liant-sérum albumine humaine, la séquence d'acides aminés du peptide liant est l'une quelconque de SEQ ID N° 11 à 16, une terminaison N du peptide liant est connectée à une terminaison C de l'analogue de GLP-1 par une liaison peptidique, et une terminaison C du peptide liant est connectée à une terminaison N de la sérum albumine humaine par une liaison peptidique, dans laquelle l'analogue de GLP-1 est l'un quelconque de ces qui suivent :
a) ayant une séquence d'acides aminés de SEQ ID N° 1 ;
b) ayant une séquence d'acides aminés de SEQ ID N° 1, dans laquelle séquence Gly à la position 2 est remplacée par Ala, Ser ou Cys et/ou Glu à la position 3 est remplacée par Asp, Gly, Ser, Cys, Thr, Asp, Gln, Tyr, Ala, Val, Ile, Leu, Met ou Phe et/ou Gly à la position 4 est remplacée par Ser, Cys, Thr, Asp, Glu, Tyr, Ala, Val, Ile, Leu, Met ou Phe et/ou Asp à la position 9 est remplacée par Glu et/ou Val à la position 10 est remplacée par Leu ou Tyr et/ou Ser à la position 12 est remplacée par Lys et/ou Glu à la position 15 est remplacée par Asp et/ou Ala à la position 18 est remplacée par Arg et/ou Lys à la position 20 est remplacée par Gly, Ser, Cys, Thr, Asp, Glu, Tyr, Ala, Val, Ile, Leu, Met, Phe ou Arg et/ou Lys à la position 28 est remplacée par Gly, Ser, Cys, Thr, Asp, Glu, Tyr, Ala, Val, Ile, Leu, Met, Phe ou Arg sans changer l'activité de celle-ci ;
c) comprenant 2 ou 3 séquences répétitives de l'analogue de GLP-1 de a) ou b), ou comprenant 2 ou 3 séquences répétitives d'un GLP-1 ; et
d) étant l'Exendine-4,
ou dans laquelle une séquence d'acides aminés de la protéine de fusion d'analogue de GLP-1 est sélectionnée parmi SEQ ID N° 3 à 5.

2. Protéine de fusion d'analogue de GLP-1 selon la revendication 1, **caractérisée en ce qu'**une séquence d'acides aminés de la sérum albumine humaine est SEQ ID N° 2 ou la séquence de SEQ ID N° 2, dans laquelle séquence Cys à la position 34 est remplacée par Ser et/ou Leu à la position 407 est remplacée par Ala et/ou Leu à la position 408 est remplacée par Val et/ou Val à la position 409 est remplacée par Ala et/ou Arg à la position 410 est remplacée par Ala et/ou Lys à la position 413 est remplacée par Gln sans changer l'activité de celle-ci.

3. Polynucléotide codant pour la protéine de fusion d'analogue de GLP-1 selon la revendication 1 ou 2.

4. Polynucléotide selon la revendication 3, **caractérisé en ce qu'**une séquence du polynucléotide est sélectionnée parmi SEQ ID N° 8 à 10.

5. Procédé de préparation de la protéine de fusion d'analogue de GLP-1 selon la revendication 1 ou 2, le procédé comprenant les étapes suivantes : construire un vecteur d'expression contenant une séquence génique de la protéine de fusion d'analogue de GLP-1, puis transformer le vecteur d'expression dans une cellule hôte pour une expression induite, et séparer et obtenir la protéine de fusion à partir de produits d'expression.

6. Procédé de préparation de la protéine de fusion d'analogue de GLP-1 selon la revendication 5, **caractérisé en ce que** le vecteur d'expression est pPIC9 ; et la cellule hôte est Pichia pastoris.

7. Procédé de préparation de la protéine de fusion d'analogue de GLP-1 selon la revendication 5, **caractérisé en ce qu'**un procédé pour séparer et obtenir la protéine de fusion à partir des produits d'expression comprend l'étape de séparation et d'obtention de la protéine de fusion en adoptant un procédé chromatographique en trois étapes qui relie chromatographie d'affinité, chromatographie hydrophobe et chromatographie par échange d'ions.

8. Protéine de fusion d'analogue de GLP-1 selon la revendication 1 ou 2 pour utilisation comme médicament.

9. Composition pharmaceutique pour utilisation dans le traitement du diabète et des maladies liées au diabète, contenant la protéine de fusion d'analogue de GLP-1 selon la revendication 1 ou 2 et au moins un vecteur ou excipient pharmaceutiquement acceptable.

10. Composition pharmaceutique pour utilisation dans le traitement du diabète et des maladies liées au diabète, contenant la protéine de fusion d'analogue de GLP-1 selon la revendication 1 ou 2 et au moins un vecteur ou excipient pharmaceutiquement acceptable.

11. Protéine de fusion d'analogue de GLP-1 selon la revendication 1 ou 2 pour utilisation dans le traitement du diabète et des maladies liées au diabète.


### SEQUENCE LIST

<110> T-mab Biopharma
<120> GLP-1 Analogue Fusion Protein and Preparation Method and Application
   thereof
<130> 130883
<160> 28
<170> PatentIn version 3.3
<210> 1
   <211> 28
   <212> PRT
   <213> Homo sapiens
<400> 1
   His Gly Glu Gly Thr Phe Thr Ser Asp Val Ser Ser Tyr Leu Glu Glu
   1               5                   10                  15
   Gln Ala Ala Lys Glu Phe Ile Ala Trp Leu Val Lys
               20                  25
<210> 2
   <211> 585
   <212> PRT
   <213> Homo sapiens
<400> 2
<210> 3
   <211> 627
   <212> PRT
   <213> Artificial
<220>
   <223> amino acid sequence of GLP-1 analogue fusion protein
<400> 3
   His Gly Glu Gly Thr Phe Thr Ser Asp Val Ser Ser Tyr Leu Glu Glu
   1               5                   10                  15
   Gln Ala Ala Lys Glu Phe Ile Ala Trp Leu Val Lys Gly Gly Gly Ser
               20                  25                  30
   Ser Pro Pro Pro Gly Gly Gly Gly Ser Ser Asp Ala His Lys Ser Glu
           35                  40                  45
   Val Ala His Arg Phe Lys Asp Leu Gly Glu Glu Asn Phe Lys Ala Leu
       50                  55                  60
   Val Leu Ile Ala Phe Ala Gln Tyr Leu Gln Gln Cys Pro Phe Glu Asp
   65                  70                  75                  80
   His Val Lys Leu Val Asn Glu Val Thr Glu Phe Ala Lys Thr Cys Val
                   85                  90                  95
   Ala Asp Glu Ser Ala Glu Asn Cys Asp Lys Ser Leu His Thr Leu Phe
               100                 105                 110
   Gly Asp Lys Leu Cys Thr Val Ala Thr Leu Arg Glu Thr Tyr Gly Glu
           115                 120                 125
   Met Ala Asp Cys Cys Ala Lys Gln Glu Pro Glu Arg Asn Glu Cys Phe
       130                 135                 140
   Leu Gln His Lys Asp Asp Asn Pro Asn Leu Pro Arg Leu Val Arg Pro
   145                 150                 155                 160
   Glu Val Asp Val Met Cys Thr Ala Phe His Asp Asn Glu Glu Thr Phe
                   165                 170                 175
   Leu Lys Lys Tyr Leu Tyr Glu Ile Ala Arg Arg His Pro Tyr Phe Tyr
               180                 185                 190
   Ala Pro Glu Leu Leu Phe Phe Ala Lys Arg Tyr Lys Ala Ala Phe Thr
           195                 200                 205
   Glu Cys Cys Gln Ala Ala Asp Lys Ala Ala Cys Leu Leu Pro Lys Leu
       210                 215                 220
   Asp Glu Leu Arg Asp Glu Gly Lys Ala Ser Ser Ala Lys Gln Arg Leu
   225                 230                 235                 240
   Lys Cys Ala Ser Leu Gln Lys Phe Gly Glu Arg Ala Phe Lys Ala Trp
                   245                 250                 255
   Ala Val Ala Arg Leu Ser Gln Arg Phe Pro Lys Ala Glu Phe Ala Glu
               260                 265                 270
   Val Ser Lys Leu Val Thr Asp Leu Thr Lys Val His Thr Glu Cys Cys
           275                 280                 285
   His Gly Asp Leu Leu Glu Cys Ala Asp Asp Arg Ala Asp Leu Ala Lys
       290                 295                 300
   Tyr Ile Cys Glu Asn Gln Asp Ser Ile Ser Ser Lys Leu Lys Glu Cys
   305                 310                 315                 320
   Cys Glu Lys Pro Leu Leu Glu Lys Ser His Cys Ile Ala Glu Val Glu
                   325                 330                 335
   Asn Asp Glu Met Pro Ala Asp Leu Pro Ser Leu Ala Ala Asp Phe Val
               340                 345                 350
   Glu Ser Lys Asp Val Cys Lys Asn Tyr Ala Glu Ala Lys Asp Val Phe
           355                 360                 365
   Leu Gly Met Phe Leu Tyr Glu Tyr Ala Arg Arg His Pro Asp Tyr Ser
       370                 375                 380
   Val Val Leu Leu Leu Arg Leu Ala Lys Thr Tyr Glu Thr Thr Leu Glu
   385                 390                 395                 400
   Lys Cys Cys Ala Ala Ala Asp Pro His Glu Cys Tyr Ala Lys Val Phe
                   405                 410                 415
   Asp Glu Phe Lys Pro Leu Val Glu Glu Pro Gln Asn Leu Ile Lys Gln
               420                 425                 430
   Asn Cys Glu Leu Phe Glu Gln Leu Gly Glu Tyr Lys Phe Gln Asn Ala
           435                 440                 445
   Leu Leu Val Arg Tyr Thr Lys Lys Val Pro Gln Val Ser Thr Pro Thr
       450                 455                 460
   Leu Val Glu Val Ser Arg Asn Leu Gly Lys Val Gly Ser Lys Cys Cys
   465                 470                 475                 480
   Lys His Pro Glu Ala Lys Arg Met Pro Cys Ala Glu Asp Tyr Leu Ser
                   485                 490                 495
   Val Val Leu Asn Gln Leu Cys Val Leu His Glu Lys Thr Pro Val Ser
               500                 505                 510
   Asp Arg Val Thr Lys Cys Cys Thr Glu Ser Leu Val Asn Arg Arg Pro
           515                 520                 525
   Cys Phe Ser Ala Leu Glu Val Asp Glu Thr Tyr Val Pro Lys Glu Phe
       530                 535                 540
   Asn Ala Glu Thr Phe Thr Phe His Ala Asp Ile Cys Thr Leu Ser Glu
   545                 550                 555                 560
   Lys Glu Arg Gln Ile Lys Lys Gln Thr Ala Leu Val Glu Leu Val Lys
                   565                 570                 575
   His Lys Pro Lys Ala Thr Lys Glu Gln Leu Lys Ala Val Met Asp Asp
               580                 585                 590
   Phe Ala Ala Phe Val Glu Lys Cys Cys Lys Ala Asp Asp Lys Glu Thr
           595                 600                 605
   Cys Phe Ala Glu Glu Gly Lys Lys Leu Val Ala Ala Ser Gln Ala Ala
       610                 615                 620
   Leu Gly Leu
   625
<210> 4
   <211> 632
   <212> PRT
   <213> Artificial
<220>
   <223> amino acid sequence of GLP-1 analogue fusion protein
<400> 4
   His Gly Glu Gly Thr Phe Thr Ser Asp Val Ser Ser Tyr Leu Glu Glu
   1               5                   10                  15
   Gln Ala Ala Lys Glu Phe Ile Ala Trp Leu Val Lys Gly Gly Gly Ala
               20                  25                  30
   Pro Pro Pro Pro Pro Pro Pro Pro Pro Pro Ser Ser Gly Gly Gly Asp
           35                  40                  45
   Ala His Lys Ser Glu Val Ala His Arg Phe Lys Asp Leu Gly Glu Glu
       50                  55                  60
   Asn Phe Lys Ala Leu Val Leu Ile Ala Phe Ala Gln Tyr Leu Gln Gln
   65                  70                  75                  80
   Cys Pro Phe Glu Asp His Val Lys Leu Val Asn Glu Val Thr Glu Phe
                   85                  90                  95
   Ala Lys Thr Cys Val Ala Asp Glu Ser Ala Glu Asn Cys Asp Lys Ser
               100                 105                 110
   Leu His Thr Leu Phe Gly Asp Lys Leu Cys Thr Val Ala Thr Leu Arg
           115                 120                 125
   Glu Thr Tyr Gly Glu Met Ala Asp Cys Cys Ala Lys Gln Glu Pro Glu
       130                 135                 140
   Arg Asn Glu Cys Phe Leu Gln His Lys Asp Asp Asn Pro Asn Leu Pro
   145                 150                 155                 160
   Arg Leu Val Arg Pro Glu Val Asp Val Met Cys Thr Ala Phe His Asp
                   165                 170                 175
   Asn Glu Glu Thr Phe Leu Lys Lys Tyr Leu Tyr Glu Ile Ala Arg Arg
               180                 185                 190
   His Pro Tyr Phe Tyr Ala Pro Glu Leu Leu Phe Phe Ala Lys Arg Tyr
           195                 200                 205
   Lys Ala Ala Phe Thr Glu Cys Cys Gln Ala Ala Asp Lys Ala Ala Cys
       210                 215                 220
   Leu Leu Pro Lys Leu Asp Glu Leu Arg Asp Glu Gly Lys Ala Ser Ser
   225                 230                 235                 240
   Ala Lys Gln Arg Leu Lys Cys Ala Ser Leu Gln Lys Phe Gly Glu Arg
                   245                 250                 255
   Ala Phe Lys Ala Trp Ala Val Ala Arg Leu Ser Gln Arg Phe Pro Lys
               260                 265                 270
   Ala Glu Phe Ala Glu Val Ser Lys Leu Val Thr Asp Leu Thr Lys Val
           275                 280                 285
   His Thr Glu Cys Cys His Gly Asp Leu Leu Glu Cys Ala Asp Asp Arg
       290                 295                 300
   Ala Asp Leu Ala Lys Tyr Ile Cys Glu Asn Gln Asp Ser Ile Ser Ser
   305                 310                 315                 320
   Lys Leu Lys Glu Cys Cys Glu Lys Pro Leu Leu Glu Lys Ser His Cys
                   325                 330                 335
   Ile Ala Glu Val Glu Asn Asp Glu Met Pro Ala Asp Leu Pro Ser Leu
               340                 345                 350
   Ala Ala Asp Phe Val Glu Ser Lys Asp Val Cys Lys Asn Tyr Ala Glu
           355                 360                 365
   Ala Lys Asp Val Phe Leu Gly Met Phe Leu Tyr Glu Tyr Ala Arg Arg
       370                 375                 380
   His Pro Asp Tyr Ser Val Val Leu Leu Leu Arg Leu Ala Lys Thr Tyr
   385                 390                 395                 400
   Glu Thr Thr Leu Glu Lys Cys Cys Ala Ala Ala Asp Pro His Glu Cys
                   405                 410                 415
   Tyr Ala Lys Val Phe Asp Glu Phe Lys Pro Leu Val Glu Glu Pro Gln
               420                 425                 430
   Asn Leu Ile Lys Gln Asn Cys Glu Leu Phe Glu Gln Leu Gly Glu Tyr
           435                 440                 445
   Lys Phe Gln Asn Ala Leu Leu Val Arg Tyr Thr Lys Lys Val Pro Gln
       450                 455                 460
   Val Ser Thr Pro Thr Leu Val Glu Val Ser Arg Asn Leu Gly Lys Val
   465                 470                 475                 480
   Gly Ser Lys Cys Cys Lys His Pro Glu Ala Lys Arg Met Pro Cys Ala
                   485                 490                 495
   Glu Asp Tyr Leu Ser Val Val Leu Asn Gln Leu Cys Val Leu His Glu
               500                 505                 510
   Lys Thr Pro Val Ser Asp Arg Val Thr Lys Cys Cys Thr Glu Ser Leu
           515                 520                 525
   Val Asn Arg Arg Pro Cys Phe Ser Ala Leu Glu Val Asp Glu Thr Tyr
       530                 535                 540
   Val Pro Lys Glu Phe Asn Ala Glu Thr Phe Thr Phe His Ala Asp Ile
   545                 550                 555                 560
   Cys Thr Leu Ser Glu Lys Glu Arg Gln Ile Lys Lys Gln Thr Ala Leu
                   565                 570                 575
   Val Glu Leu Val Lys His Lys Pro Lys Ala Thr Lys Glu Gln Leu Lys
               580                 585                 590
   Ala Val Met Asp Asp Phe Ala Ala Phe Val Glu Lys Cys Cys Lys Ala
           595                 600                 605
   Asp Asp Lys Glu Thr Cys Phe Ala Glu Glu Gly Lys Lys Leu Val Ala
       610                 615                 620
   Ala Ser Gln Ala Ala Leu Gly Leu
   625                 630
<210> 5
   <211> 639
   <212> PRT
   <213> Artificial
<220>
   <223> amino acid sequence of GLP-1 analogue fusion protein
<400> 5
   His Gly Glu Gly Thr Phe Thr Ser Asp Val Ser Ser Tyr Leu Glu Glu
   1               5                   10                  15
   Gln Ala Ala Lys Glu Phe Ile Ala Trp Leu Val Lys Gly Gly Gly Ser
               20                  25                  30
   Ser Gly Ala Pro Pro Pro Ser Gly Gly Gly Gly Ser Gly Gly Gly Gly
           35                  40                  45
   Ser Gly Gly Gly Gly Ser Asp Ala His Lys Ser Glu Val Ala His Arg
       50                  55                  60
   Phe Lys Asp Leu Gly Glu Glu Asn Phe Lys Ala Leu Val Leu Ile Ala
   65                  70                  75                  80
   Phe Ala Gln Tyr Leu Gln Gln Cys Pro Phe Glu Asp His Val Lys Leu
                   85                  90                  95
   Val Asn Glu Val Thr Glu Phe Ala Lys Thr Cys Val Ala Asp Glu Ser
               100                 105                 110
   Ala Glu Asn Cys Asp Lys Ser Leu His Thr Leu Phe Gly Asp Lys Leu
           115                 120                 125
   Cys Thr Val Ala Thr Leu Arg Glu Thr Tyr Gly Glu Met Ala Asp Cys
       130                 135                 140
   Cys Ala Lys Gln Glu Pro Glu Arg Asn Glu Cys Phe Leu Gln His Lys
   145                 150                 155                 160
   Asp Asp Asn Pro Asn Leu Pro Arg Leu Val Arg Pro Glu Val Asp Val
                   165                 170                 175
   Met Cys Thr Ala Phe His Asp Asn Glu Glu Thr Phe Leu Lys Lys Tyr
               180                 185                 190
   Leu Tyr Glu Ile Ala Arg Arg His Pro Tyr Phe Tyr Ala Pro Glu Leu
           195                 200                 205
   Leu Phe Phe Ala Lys Arg Tyr Lys Ala Ala Phe Thr Glu Cys Cys Gln
       210                 215                 220
   Ala Ala Asp Lys Ala Ala Cys Leu Leu Pro Lys Leu Asp Glu Leu Arg
   225                 230                 235                 240
   Asp Glu Gly Lys Ala Ser Ser Ala Lys Gln Arg Leu Lys Cys Ala Ser
                   245                 250                 255
   Leu Gln Lys Phe Gly Glu Arg Ala Phe Lys Ala Trp Ala Val Ala Arg
               260                 265                 270
   Leu Ser Gln Arg Phe Pro Lys Ala Glu Phe Ala Glu Val Ser Lys Leu
           275                 280                 285
   Val Thr Asp Leu Thr Lys Val His Thr Glu Cys Cys His Gly Asp Leu
       290                 295                 300
   Leu Glu Cys Ala Asp Asp Arg Ala Asp Leu Ala Lys Tyr Ile Cys Glu
   305                 310                 315                 320
   Asn Gln Asp Ser Ile Ser Ser Lys Leu Lys Glu Cys Cys Glu Lys Pro
                   325                 330                 335
   Leu Leu Glu Lys Ser His Cys Ile Ala Glu Val Glu Asn Asp Glu Met
               340                 345                 350
   Pro Ala Asp Leu Pro Ser Leu Ala Ala Asp Phe Val Glu Ser Lys Asp
           355                 360                 365
   Val Cys Lys Asn Tyr Ala Glu Ala Lys Asp Val Phe Leu Gly Met Phe
       370                 375                 380
   Leu Tyr Glu Tyr Ala Arg Arg His Pro Asp Tyr Ser Val Val Leu Leu
   385                 390                 395                 400
   Leu Arg Leu Ala Lys Thr Tyr Glu Thr Thr Leu Glu Lys Cys Cys Ala
                   405                 410                 415
   Ala Ala Asp Pro His Glu Cys Tyr Ala Lys Val Phe Asp Glu Phe Lys
               420                 425                 430
   Pro Leu Val Glu Glu Pro Gln Asn Leu Ile Lys Gln Asn Cys Glu Leu
           435                 440                 445
   Phe Glu Gln Leu Gly Glu Tyr Lys Phe Gln Asn Ala Leu Leu Val Arg
       450                 455                 460
   Tyr Thr Lys Lys Val Pro Gln Val Ser Thr Pro Thr Leu Val Glu Val
   465                 470                 475                 480
   Ser Arg Asn Leu Gly Lys Val Gly Ser Lys Cys Cys Lys His Pro Glu
                   485                 490                 495
   Ala Lys Arg Met Pro Cys Ala Glu Asp Tyr Leu Ser Val Val Leu Asn
               500                 505                 510
   Gln Leu Cys Val Leu His Glu Lys Thr Pro Val Ser Asp Arg Val Thr
           515                 520                 525
   Lys Cys Cys Thr Glu Ser Leu Val Asn Arg Arg Pro Cys Phe Ser Ala
       530                 535                 540
   Leu Glu Val Asp Glu Thr Tyr Val Pro Lys Glu Phe Asn Ala Glu Thr
   545                 550                 555                 560
   Phe Thr Phe His Ala Asp Ile Cys Thr Leu Ser Glu Lys Glu Arg Gln
                   565                 570                 575
   Ile Lys Lys Gln Thr Ala Leu Val Glu Leu Val Lys His Lys Pro Lys
               580                 585                 590
   Ala Thr Lys Glu Gln Leu Lys Ala Val Met Asp Asp Phe Ala Ala Phe
           595                 600                 605
   Val Glu Lys Cys Cys Lys Ala Asp Asp Lys Glu Thr Cys Phe Ala Glu
       610                 615                 620
   Glu Gly Lys Lys Leu Val Ala Ala Ser Gln Ala Ala Leu Gly Leu
   625                 630                 635
<210> 6
   <211> 84
   <212> DNA
   <213> Homo sapiens
<400> 6
   cacggcgaag ggacctttac cagtgatgta agttcttatt tggaagagca agctgccaag      60
   gaattcattg cttggctggt gaaa                                             84
<210> 7
   <211> 1758
   <212> DNA
   <213> Homo sapiens
<400> 7
   gatgcacaca agagtgaggt tgctcatcgg tttaaagatt tgggagaaga aaatttcaaa     60
   gccttggtgt tgattgcctt tgctcagtat cttcagcagt gtccatttga agatcatgta    120
   aaattagtga atgaagtaac tgaatttgca aaaacatgtg ttgctgatga gtcagctgaa    180
   aattgtgaca aatcacttca tacccttttt ggagacaaat tatgcacagt tgcaactctt    240
   cgtgaaacct atggtgaaat ggctgactgc tgtgcaaaac aagaacctga gagaaatgaa    300
   tgcttcttgc aacacaaaga tgacaaccca aacctccccc gattggtgag accagaggtt    360
   gatgtgatgt gcactgcttt tcatgacaat gaagagacat ttttgaaaaa atacttatat    420
   gaaattgcca gaagacatcc ttacttttat gccccggaac tccttttctt tgctaaaagg    480
   tataaagctg cttttacaga atgttgccaa gctgctgata aagctgcctg cctgttgcca    540
   aagctcgatg aacttcggga tgaagggaag gcttcgtctg ccaaacagag actcaagtgt    600
   gccagtctcc aaaaatttgg agaaagagct ttcaaagcat gggcagtagc tcgcctgagc    660
   cagagatttc ccaaagctga gtttgcagaa gtttccaagt tagtgacaga tcttaccaaa    720
   gtccacacgg aatgctgcca tggagatctg cttgaatgtg ctgatgacag ggcggacctt    780
   gccaagtata tctgtgaaaa tcaagattcg atctccagta aactgaagga atgctgtgaa    840
   aaacctctgt tggaaaaatc ccactgcatt gccgaagtgg aaaatgatga gatgcctgct    900
   gacttgcctt cattagctgc tgattttgtt gaaagtaagg atgtttgcaa aaactatgct    960
   gaggcaaagg atgtcttcct gggcatgttt ttgtatgaat atgcaagaag gcatcctgat   1020
   tactctgtcg tgctgctgct gagacttgcc aagacatatg aaaccactct agagaagtgc   1080
   tgtgccgctg cagatcctca tgaatgctat gccaaagtgt tcgatgaatt taaacctctt   1140
   gtggaagagc ctcagaattt aatcaaacaa aattgtgagc tttttgagca gcttggagag   1200
   tacaaattcc agaatgcgct attagttcgt tacaccaaga aagtacccca agtgtcaact   1260
   ccaactcttg tagaggtctc aagaaaccta ggaaaagtgg gcagcaaatg ttgtaaacat   1320
   cctgaagcaa aaagaatgcc ctgtgcagaa gactatctat ccgtggtcct gaaccagtta   1380
   tgtgtgttgc atgagaaaac gccagtaagt gacagagtca caaaatgctg cacagagtcc   1440
   ttggtgaaca ggcgaccatg cttttcagct ctggaagtcg atgaaacata cgttcccaaa   1500
   gagtttaatg ctgaaacatt caccttccat gcagatatat gcacactttc tgagaaggag   1560
   agacaaatca agaaacaaac tgcacttgtt gagcttgtga aacacaagcc caaggcaaca   1620
   aaagagcaac tgaaagctgt tatggatgat ttcgcagctt ttgtagagaa gtgctgcaag   1680
   gctgacgata aggagacctg ctttgccgag gagggtaaaa aacttgttgc tgcaagtcaa   1740
   gctgccttag gcttataa                                                 1758
<210> 8
   <211> 1884
   <212> DNA
   <213> Artificial
<220>
   <223> nucleotide coding sequence of GLP-1 analogue fusion protein
<400> 8
   cacggcgaag ggacctttac cagtgatgta agttcttatt tggaagagca agctgccaag      60
   gaattcattg cttggctggt gaaaggtggt ggatcttctc caccaccagg tggtggaggc     120
   tcttcagatg cacacaagag tgaggttgct catcggttta aagatttggg agaagaaaat     180
   ttcaaagcct tggtgttgat tgcctttgct cagtatcttc agcagtgtcc atttgaagat     240
   catgtaaaat tagtgaatga agtaactgaa tttgcaaaaa catgtgttgc tgatgagtca     300
   gctgaaaatt gtgacaaatc acttcatacc ctttttggag acaaattatg cacagttgca     360
   actcttcgtg aaacctatgg tgaaatggct gactgctgtg caaaacaaga acctgagaga     420
   aatgaatgct tcttgcaaca caaagatgac aacccaaacc tcccccgatt ggtgagacca     480
   gaggttgatg tgatgtgcac tgcttttcat gacaatgaag agacattttt gaaaaaatac     540
   ttatatgaaa ttgccagaag acatccttac ttttatgccc cggaactcct tttctttgct     600
   aaaaggtata aagctgcttt tacagaatgt tgccaagctg ctgataaagc tgcctgcctg     660
   ttgccaaagc tcgatgaact tcgggatgaa gggaaggctt cgtctgccaa acagagactc     720
   aagtgtgcca gtctccaaaa atttggagaa agagctttca aagcatgggc agtagctcgc     780
   ctgagccaga gatttcccaa agctgagttt gcagaagttt ccaagttagt gacagatctt     840
   accaaagtcc acacggaatg ctgccatgga gatctgcttg aatgtgctga tgacagggcg     900
   gaccttgcca agtatatctg tgaaaatcaa gattcgatct ccagtaaact gaaggaatgc     960
   tgtgaaaaac ctctgttgga aaaatcccac tgcattgccg aagtggaaaa tgatgagatg    1020
   cctgctgact tgccttcatt agctgctgat tttgttgaaa gtaaggatgt ttgcaaaaac    1080
   tatgctgagg caaaggatgt cttcctgggc atgtttttgt atgaatatgc aagaaggcat    1140
   cctgattact ctgtcgtgct gctgctgaga cttgccaaga catatgaaac cactctagag    1200
   aagtgctgtg ccgctgcaga tcctcatgaa tgctatgcca aagtgttcga tgaatttaaa    1260
   cctcttgtgg aagagcctca gaatttaatc aaacaaaatt gtgagctttt tgagcagctt    1320
   ggagagtaca aattccagaa tgcgctatta gttcgttaca ccaagaaagt accccaagtg    1380
   tcaactccaa ctcttgtaga ggtctcaaga aacctaggaa aagtgggcag caaatgttgt    1440
   aaacatcctg aagcaaaaag aatgccctgt gcagaagact atctatccgt ggtcctgaac    1500
   cagttatgtg tgttgcatga gaaaacgcca gtaagtgaca gagtcacaaa atgctgcaca    1560
   gagtccttgg tgaacaggcg accatgcttt tcagctctgg aagtcgatga aacatacgtt    1620
   cccaaagagt ttaatgctga aacattcacc ttccatgcag atatatgcac actttctgag    1680
   aaggagagac aaatcaagaa acaaactgca cttgttgagc ttgtgaaaca caagcccaag    1740
   gcaacaaaag agcaactgaa agctgttatg gatgatttcg cagcttttgt agagaagtgc    1800
   tgcaaggctg acgataagga gacctgcttt gccgaggagg gtaaaaaact tgttgctgca    1860
   agtcaagctg ccttaggctt ataa                                           1884
<210> 9
   <211> 1899
   <212> DNA
   <213> Artificial
<220>
   <223> nucleotide coding sequence of GLP-1 analogue fusion protein
<400> 9
   cacggcgaag ggacctttac cagtgatgta agttcttatt tggaagagca agctgccaag     60
   gaattcattg cttggctggt gaaaggcggg ggtgctccac caccaccacc accaccacca    120
   ccaccatctt ccggaggcgg tgatgcacac aagagtgagg ttgctcatcg gtttaaagat    180
   ttgggagaag aaaatttcaa agccttggtg ttgattgcct ttgctcagta tcttcagcag    240
   tgtccatttg aagatcatgt aaaattagtg aatgaagtaa ctgaatttgc aaaaacatgt    300
   gttgctgatg agtcagctga aaattgtgac aaatcacttc ataccctttt tggagacaaa    360
   ttatgcacag ttgcaactct tcgtgaaacc tatggtgaaa tggctgactg ctgtgcaaaa    420
   caagaacctg agagaaatga atgcttcttg caacacaaag atgacaaccc aaacctcccc    480
   cgattggtga gaccagaggt tgatgtgatg tgcactgctt ttcatgacaa tgaagagaca    540
   tttttgaaaa aatacttata tgaaattgcc agaagacatc cttactttta tgccccggaa    600
   ctccttttct ttgctaaaag gtataaagct gcttttacag aatgttgcca agctgctgat    660
   aaagctgcct gcctgttgcc aaagctcgat gaacttcggg atgaagggaa ggcttcgtct    720
   gccaaacaga gactcaagtg tgccagtctc caaaaatttg gagaaagagc tttcaaagca    780
   tgggcagtag ctcgcctgag ccagagattt cccaaagctg agtttgcaga agtttccaag    840
   ttagtgacag atcttaccaa agtccacacg gaatgctgcc atggagatct gcttgaatgt    900
   gctgatgaca gggcggacct tgccaagtat atctgtgaaa atcaagattc gatctccagt    960
   aaactgaagg aatgctgtga aaaacctctg ttggaaaaat cccactgcat tgccgaagtg   1020
   gaaaatgatg agatgcctgc tgacttgcct tcattagctg ctgattttgt tgaaagtaag   1080
   gatgtttgca aaaactatgc tgaggcaaag gatgtcttcc tgggcatgtt tttgtatgaa   1140
   tatgcaagaa ggcatcctga ttactctgtc gtgctgctgc tgagacttgc caagacatat   1200
   gaaaccactc tagagaagtg ctgtgccgct gcagatcctc atgaatgcta tgccaaagtg   1260
   ttcgatgaat ttaaacctct tgtggaagag cctcagaatt taatcaaaca aaattgtgag   1320
   ctttttgagc agcttggaga gtacaaattc cagaatgcgc tattagttcg ttacaccaag   1380
   aaagtacccc aagtgtcaac tccaactctt gtagaggtct caagaaacct aggaaaagtg   1440
   ggcagcaaat gttgtaaaca tcctgaagca aaaagaatgc cctgtgcaga agactatcta   1500
   tccgtggtcc tgaaccagtt atgtgtgttg catgagaaaa cgccagtaag tgacagagtc   1560
   acaaaatgct gcacagagtc cttggtgaac aggcgaccat gcttttcagc tctggaagtc   1620
   gatgaaacat acgttcccaa agagtttaat gctgaaacat tcaccttcca tgcagatata   1680
   tgcacacttt ctgagaagga gagacaaatc aagaaacaaa ctgcacttgt tgagcttgtg   1740
   aaacacaagc ccaaggcaac aaaagagcaa ctgaaagctg ttatggatga tttcgcagct   1800
   tttgtagaga agtgctgcaa ggctgacgat aaggagacct gctttgccga ggagggtaaa   1860
   aaacttgttg ctgcaagtca agctgcctta ggcttataa                          1899
<210> 10
   <211> 1920
   <212> DNA
   <213> Artificial
<220>
   <223> nucleotide coding sequence of GLP-1 analogue fusion protein
<400> 10
   cacggcgaag ggacctttac cagtgatgta agttcttatt tggaagagca agctgccaag     60
   gaattcattg cttggctggt gaaaggcggt ggatcttctg gtgctccacc accatctggt    120
   ggtggaggct ctggaggtgg aggttccgga ggcgggggtt cagatgcaca caagagtgag    180
   gttgctcatc ggtttaaaga tttgggagaa gaaaatttca aagccttggt gttgattgcc    240
   tttgctcagt atcttcagca gtgtccattt gaagatcatg taaaattagt gaatgaagta    300
   actgaatttg caaaaacatg tgttgctgat gagtcagctg aaaattgtga caaatcactt    360
   catacccttt ttggagacaa attatgcaca gttgcaactc ttcgtgaaac ctatggtgaa    420
   atggctgact gctgtgcaaa acaagaacct gagagaaatg aatgcttctt gcaacacaaa    480
   gatgacaacc caaacctccc ccgattggtg agaccagagg ttgatgtgat gtgcactgct    540
   tttcatgaca atgaagagac atttttgaaa aaatacttat atgaaattgc cagaagacat    600
   ccttactttt atgccccgga actccttttc tttgctaaaa ggtataaagc tgcttttaca    660
   gaatgttgcc aagctgctga taaagctgcc tgcctgttgc caaagctcga tgaacttcgg    720
   gatgaaggga aggcttcgtc tgccaaacag agactcaagt gtgccagtct ccaaaaattt    780
   ggagaaagag ctttcaaagc atgggcagta gctcgcctga gccagagatt tcccaaagct    840
   gagtttgcag aagtttccaa gttagtgaca gatcttacca aagtccacac ggaatgctgc    900
   catggagatc tgcttgaatg tgctgatgac agggcggacc ttgccaagta tatctgtgaa    960
   aatcaagatt cgatctccag taaactgaag gaatgctgtg aaaaacctct gttggaaaaa   1020
   tcccactgca ttgccgaagt ggaaaatgat gagatgcctg ctgacttgcc ttcattagct   1080
   gctgattttg ttgaaagtaa ggatgtttgc aaaaactatg ctgaggcaaa ggatgtcttc   1140
   ctgggcatgt ttttgtatga atatgcaaga aggcatcctg attactctgt cgtgctgctg   1200
   ctgagacttg ccaagacata tgaaaccact ctagagaagt gctgtgccgc tgcagatcct   1260
   catgaatgct atgccaaagt gttcgatgaa tttaaacctc ttgtggaaga gcctcagaat   1320
   ttaatcaaac aaaattgtga gctttttgag cagcttggag agtacaaatt ccagaatgcg   1380
   ctattagttc gttacaccaa gaaagtaccc caagtgtcaa ctccaactct tgtagaggtc   1440
   tcaagaaacc taggaaaagt gggcagcaaa tgttgtaaac atcctgaagc aaaaagaatg   1500
   ccctgtgcag aagactatct atccgtggtc ctgaaccagt tatgtgtgtt gcatgagaaa   1560
   acgccagtaa gtgacagagt cacaaaatgc tgcacagagt ccttggtgaa caggcgacca   1620
   tgcttttcag ctctggaagt cgatgaaaca tacgttccca aagagtttaa tgctgaaaca   1680
   ttcaccttcc atgcagatat atgcacactt tctgagaagg agagacaaat caagaaacaa   1740
   actgcacttg ttgagcttgt gaaacacaag cccaaggcaa caaaagagca actgaaagct   1800
   gttatggatg atttcgcagc ttttgtagag aagtgctgca aggctgacga taaggagacc   1860
   tgctttgccg aggagggtaa aaaacttgtt gctgcaagtc aagctgcctt aggcttataa   1920
<210> 11
   <211> 14
   <212> PRT
   <213> Artificial
<220>
   <223> connecting peptide
<400> 11
   Gly Gly Gly Ser Ser Pro Pro Pro Gly Gly Gly Gly Ser Ser
   1               5                   10
<210> 12
   <211> 24
   <212> PRT
   <213> Artificial
<220>
   <223> connecting peptide
<400> 12
   Gly Gly Gly Ser Ser Gly Gly Gly Ser Ser Pro Pro Pro Ala Gly Gly
   1               5                   10                  15
   Gly Ser Ser Gly Gly Gly Ser Ser
               20
<210> 13
   <211> 19
   <212> PRT
   <213> Artificial
<220>
   <223> connecting peptide
<400> 13
   Gly Gly Gly Ala Pro Pro Pro Pro Pro Pro Pro Pro Pro Pro Ser Ser
   1               5                   10                  15
   Gly Gly Gly
<210> 14
   <211> 24
   <212> PRT
   <213> Artificial
<220>
   <223> connecting peptide
<400> 14
   Ala Gly Gly Gly Ala Ala Gly Gly Gly Ser Ser Gly Gly Gly Pro Pro
   1               5                   10                  15
   Pro Pro Pro Gly Gly Gly Gly Ser
               20
<210> 15
   <211> 15
   <212> PRT
   <213> Artificial
<220>
   <223> connecting peptide
<400> 15
   Gly Gly Ser Ser Gly Ala Pro Pro Pro Pro Gly Gly Gly Gly Ser
   1               5                   10                  15
<210> 16
   <211> 26
   <212> PRT
   <213> Artificial
<220>
   <223> connecting peptide
<400> 16
   Gly Gly Gly Ser Ser Gly Ala Pro Pro Pro Ser Gly Gly Gly Gly Ser
   1               5                   10                  15
   Gly Gly Gly Gly Ser Gly Gly Gly Gly Ser
               20                  25
<210> 17
   <211> 187
   <212> DNA
   <213> Artificial
<220>
   <223> gene segment of (GLP-1 analogue)2 with an HSA fusion segment at
   3\222-terminal
<400> 17
   cacggcgaag ggacctttac cagtgatgta agttcttatt tggaagagca agctgccaag     60
   gaattcattg cttggctggt gaaacacggc gaagggacct ttaccagtga tgtaagttct    120
   tatttggaag agcaagctgc caaggaattc attgcttggc tggtgaaaga tgcacacaag    180
   agtgagg                                                              187
<210> 18
   <211> 148
   <212> DNA
   <213> Artificial
<220>
   <223> gene segment of (GLP-1 analogue)- (Gly4Ser)3 with an HSA fusion
   segment at 3\222-terminal
<400> 18
   cacggcgaag ggacctttac cagtgatgta agttcttatt tggaagagca agctgccaag     60
   gaattcattg cttggctggt gaaaggtggt ggaggctctg gaggtggagg ttccggaggc    120
   gggggttcag atgcacacaa gagtgagg                                       148
<210> 19
   <211> 163
   <212> DNA
   <213> Artificial
<220>
   <223> gene segment of (GLP-1 analogue)- (Gly4Ser)4 with an HSA fusion
   segment at 3\222-terminal
<400> 19
   cacggcgaag ggacctttac cagtgatgta agttcttatt tggaagagca agctgccaag     60
   gaattcattg cttggctggt gaaaggtggt ggaggctctg gtggtggagg ctctggaggt    120
   ggaggttccg gaggcggggg ttcagatgca cacaagagtg agg                      163
<210> 20
   <211> 145
   <212> DNA
   <213> Artificial
<220>
   <223> gene segment of (GLP-1 analogue)-El with an HSA fusion segment at
   3\222-terminal
<400> 20
   cacggcgaag ggacctttac cagtgatgta agttcttatt tggaagagca agctgccaag     60
   gaattcattg cttggctggt gaaaggtggt ggatcttctc caccaccagg tggtggaggc    120
   tcttcagatg cacacaagag tgagg                                          145
<210> 21
   <211> 175
   <212> DNA
   <213> Artificial
<220>
   <223> gene segment of (GLP-1 analogue)-E2 with an HSA fusion segment at
   3\222-terminal
<400> 21
   cacggcgaag ggacctttac cagtgatgta agttcttatt tggaagagca agctgccaag     60
   gaattcattg cttggctggt gaaaggtgga ggctcttcag gtggaggctc ttcaccacca    120
   ccagctggtg gaggctcttc aggtggaggc tcttcagatg cacacaagag tgagg         175
<210> 22
   <211> 160
   <212> DNA
   <213> Artificial
<220>
   <223> gene segment of (GLP-1 analogue)-E3 with an HSA fusion segment at
   3\222-terminal
<400> 22
   cacggcgaag ggacctttac cagtgatgta agttcttatt tggaagagca agctgccaag     60
   gaattcattg cttggctggt gaaaggcggg ggtgctccac caccaccacc accaccacca    120
   ccaccatctt ccggaggcgg tgatgcacac aagagtgagg                          160
<210> 23
   <211> 175
   <212> DNA
   <213> Artificial
<220>
   <223> gene segment of (GLP-1 analogue)-E4 with an HSA fusion segment at
   3\222-terminal
<400> 23
   cacggcgaag ggacctttac cagtgatgta agttcttatt tggaagagca agctgccaag     60
   gaattcattg cttggctggt gaaagctggc gggggtgctg ctggaggcgg gtcttctggc    120
   gggggtccac caccaccacc aggaggcggg ggttcagatg cacacaagag tgagg         175
<210> 24
   <211> 148
   <212> DNA
   <213> Artificial
<220>
   <223> gene segment of (GLP-1 analogue)-E5 with an HSA fusion segment at
   3\222-terminal
<400> 24
   cacggcgaag ggacctttac cagtgatgta agttcttatt tggaagagca agctgccaag     60
   gaattcattg cttggctggt gaaaggtgga tcttctggtg ctccaccacc accaggaggc    120
   gggggttcag atgcacacaa gagtgagg                                       148
<210> 25
   <211> 181
   <212> DNA
   <213> Artificial
<220>
   <223> gene segment of (GLP-1 analogue)-E6 with an HSA fusion segment at
   3\222-terminal
<400> 25
   cacggcgaag ggacctttac cagtgatgta agttcttatt tggaagagca agctgccaag     60
   gaattcattg cttggctggt gaaaggcggt ggatcttctg gtgctccacc accatctggt    120
   ggtggaggct ctggaggtgg aggttccgga ggcgggggtt cagatgcaca caagagtgag    180
   g                                                                    181
<210> 26
   <211> 40
   <212> DNA
   <213> Artificial
<220>
   <223> primer
<400> 26
   tctctcgaga aaagacacgg cgaagggacc tttaccagtg 40
<210> 27
   <211> 19
   <212> DNA
   <213> Artificial
<220>
   <223> primer
<400> 27
   gatgcacaca agagtgagg 19
<210> 28
   <211> 32
   <212> DNA
   <213> Artificial
<220>
   <223> primer
<400> 28
   ttagcggccg cttataagcc taaggcagct tg 32